(19) Europäisches Patentamt
European Patent Office
Office européen des brevets

(11) EP 3 872 184 A1

(12) EUROPEAN PATENT APPLICATION
published in accordance with Art. 153(4) EPC

(43) Date of publication:
**01.09.2021 Bulletin 2021/35**

(21) Application number: **19876614.9**

(22) Date of filing: **25.10.2019**

(51) Int Cl.:
***C12P 19/04*** *(2006.01)* ***C08K 3/24*** *(2006.01)*
***C08L 1/02*** *(2006.01)* ***C08L 89/00*** *(2006.01)*
***D21H 11/20*** *(2006.01)*

(86) International application number:
**PCT/JP2019/041876**

(87) International publication number:
**WO 2020/085479 (30.04.2020 Gazette 2020/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.10.2018 JP 2018202334**

(71) Applicant: **Oji Holdings Corporation**
**Chuo-ku**
**Tokyo 104-0061 (JP)**

(72) Inventors:
- **MIZUKAMI, Moe**
  **Tokyo 104-0061 (JP)**
- **FUSHIMI, Hayato**
  **Tokyo 104-0061 (JP)**
- **SUNAGAWA, Hirokazu**
  **Tokyo 104-0061 (JP)**

(74) Representative: **Godemeyer Blum Lenze Patentanwälte**
**Partnerschaft mbB - werkpatent**
**An den Gärten 7**
**51491 Overath (DE)**

# (54) FINE FIBROUS CELLULOSE-CONTAINING COMPOSITION AND METHOD FOR MANUFACTURING SAME

(57) Provided are a fine fibrous cellulose-containing composition, and a method for manufacturing the same, which are capable of reducing the viscosity of a fine fibrous cellulose dispersion liquid without changing the concentration of fine fibrous cellulose. The fine fibrous cellulose-containing composition contains fine fibrous cellulose having a fiber width of 1,000 nm or less, an enzyme-derived protein, and a protease or a chlorine-containing oxo acid or a salt thereof.

EP 3 872 184 A1

## Description

### Technical Field

The present invention relates to fine fibrous cellulose and a method for manufacturing the same.

### Background Art

Fine fibrous cellulose having a fiber width of 1,000 nm or less is called cellulose nanofiber, and is expected to be expanded for various fields such as a reinforcing material for a composite material, a high-performance filter, a display or a solar cell, electronics, transportation equipment, building materials, and a medical treatment.

In general, the fine fibrous cellulose is obtained as a dispersion liquid. However, the corresponding dispersion liquid has a high viscosity. For example, in forming a film on a base material, when the concentration of the dispersion liquid is too high, homogeneous coating is difficult. Meanwhile, when the dispersion liquid is diluted in use for homogeneous coating, coating and drying have to be repeatedly performed many times until a desired film thickness is achieved, thereby causing a problem of inefficiency.

PTL 1 discloses a method of producing a low-viscosity cellulose nanofiber dispersion liquid. In the method, wood from which pulp having a specific fiber length distribution is obtained is selected, and the pulp derived from the corresponding wood is defibrated to obtain the cellulose nanofiber dispersion liquid.

### Citation List

### Patent Literature

PTL 1: JP2018-090949 A

### Summary of Invention

### Technical Problem

In the method described in PTL 1, it is necessary to use a specific raw material, and also, it was difficult to perform a precise viscosity adjustment for the obtained dispersion liquid.

An object of the present invention is to provide a fine fibrous cellulose-containing composition, and a production method thereof, which are capable of reducing the viscosity of a fine fibrous cellulose dispersion liquid without changing the concentration of fine fibrous cellulose, and are also excellent in the stability of the viscosity. Further, an object is to provide a molded product containing the fine fibrous cellulose-containing composition.

### Solution to Problem

The present inventors have found that the above-mentioned problems are solved by a composition containing fine fibrous cellulose, an enzyme-derived protein, and a chlorine-containing oxo acid or a salt thereof.

That is, the present invention relates to followings <1> to <17>.

<1> A fine fibrous cellulose-containing composition containing fine fibrous cellulose having a fiber width of 1,000 nm or less, a protein derived from an enzyme, and a protease or a chlorine-containing oxo acid or a salt thereof.

<2> The fine fibrous cellulose-containing composition described in <1>, wherein the enzyme is a cellulase.

<3> The fine fibrous cellulose-containing composition described in <1> or <2>, wherein a degree of polymerization of the fine fibrous cellulose is 430 or less.

<4> The fine fibrous cellulose-containing composition described in any one of <1> to <3>, wherein the fine fibrous cellulose has an ionic substituent, and a content of the ionic substituent is 0.60 mmol/g or more with respect to the fine fibrous cellulose.

<5> The fine fibrous cellulose-containing composition described in <4>, wherein the ionic substituent is at least one selected from a carboxymethyl group, a sulfonic acid group, a group derived from the sulfonic acid group, a phosphoric acid group, a group derived from the phosphoric acid group, a carboxy group, and a group derived from the carboxy group.

<6> The fine fibrous cellulose-containing composition described in any one of <1> to <5>, wherein the fine fibrous cellulose-containing composition contains a chlorine-containing oxo acid or a salt thereof, and a content of the chlorine-containing oxo acid or a salt thereof is 0.0001 parts by mass or more and 10 parts by mass or less based

on 100 parts by mass of the fine fibrous cellulose.

<7> The fine fibrous cellulose-containing composition described in any one of <1> to <6>, wherein the fine fibrous cellulose-containing composition contains a chlorine-containing oxo acid or a salt thereof, and contains 10 parts by mass or more and 1,000,000 parts by mass or less of the chlorine-containing oxo acid or a salt thereof based on 100 parts by mass of the protein derived from the enzyme.

<8> The fine fibrous cellulose-containing composition described in any one of <1> to <7>, wherein the fine fibrous cellulose-containing composition contains a chlorine-containing oxo acid or a salt thereof, and the chlorine-containing oxo acid or a salt thereof includes at least one selected from the group consisting of sodium hypochlorite, potassium hypochlorite, and calcium hypochlorite.

<9> The fine fibrous cellulose-containing composition described in any one of <1> to <5>, wherein the fine fibrous cellulose-containing composition contains a protease, and a content of the protease is 0.0002 U or more and 20 U or less based on 1 mg of the fine fibrous cellulose.

<10> The fine fibrous cellulose-containing composition described in any one of <1> to <5>, and <9>, wherein the fine fibrous cellulose-containing composition contains a protease, and contains 1 U or more and 1,000,000 U or less of the protease based on 1 mg of the protein derived from the enzyme.

<11> The fine fibrous cellulose-containing composition described in any one of <1> to <10>, wherein when a solid content concentration of the fine fibrous cellulose is 0.4% by mass, a composition viscosity is $1.0 \times 10^4$ mPa·s or less as measured by using a B-type viscometer at 23°C through rotation for 3 min at a rotation speed of 3 rpm.

<12> The fine fibrous cellulose-containing composition described in any one of <1> to <11>, which has a slurry form.

<13> The fine fibrous cellulose-containing composition described in any one of <1> to <10>, which has a powder form.

<14> The fine fibrous cellulose-containing composition described in any one of <1> to <10>, which has a sheet form.

<15> A method of producing a fine fibrous cellulose-containing composition, the method including, in this order: defibering fibrous cellulose in a solvent to obtain a fine fibrous cellulose-containing dispersion liquid; adding an enzyme to the fine fibrous cellulose-containing dispersion liquid; and adding a protease, or a chlorine-containing oxo acid or a salt thereof to the fine fibrous cellulose-containing dispersion liquid treated with the enzyme.

<16> The method of producing the fine fibrous cellulose-containing composition described in <15>, wherein the enzyme is a cellulase, and an addition amount of the cellulase is 0.0001 U or more and 1.0 U or less based on 1 mg of the fine fibrous cellulose.

<17> The method of producing the fine fibrous cellulose-containing composition described in <15> or <16>, wherein the fibrous cellulose has an ionic substituent.

## Advantageous Effects of Invention

According to the present invention, it is possible to provide a fine fibrous cellulose-containing composition, and a production method thereof, which are capable of reducing the viscosity of a fine fibrous cellulose dispersion liquid without changing the concentration of fine fibrous cellulose, and are also excellent in the stability of the viscosity. Further, it is possible to provide a molded product containing the fine fibrous cellulose-containing composition.

## Brief Description of Drawings

Fig. 1 is a graph illustrating the relationship between the electrical conductivity and the amount of NaOH added dropwise to fine fibrous cellulose having a phosphoric acid group; and

Fig. 2 is a graph illustrating the relationship between the electrical conductivity and the amount of NaOH added dropwise to fine fibrous cellulose having a carboxy group.

## Description of Embodiments

### [Fine Fibrous Cellulose-containing composition]

A fine fibrous cellulose-containing composition of the present invention contains fine fibrous cellulose having a fiber width of 1,000 nm or less, an enzyme-derived protein, and a protease or a chlorine-containing oxo acid or a salt thereof. That is, a first fine fibrous cellulose-containing composition of the present invention contains fine fibrous cellulose, an enzyme-derived protein, and a protease, and a second fine fibrous cellulose-containing composition of the present invention contains fine fibrous cellulose, an enzyme-derived protein, and a chlorine-containing oxo acid or a salt thereof.

As a method of reducing the viscosity of a dispersion liquid of fine fibrous cellulose, a method of increasing the number of defibering steps has conventionally been exemplified. However, in the viscosity reduction through the defibering process, it is difficult to perform a precise viscosity control, and also, the defibering process has a large burden in time and cost.

In the fine fibrous cellulose-containing composition of the present invention, it is possible to reduce the viscosity of a fine fibrous cellulose dispersion liquid without changing the concentration of fine fibrous cellulose, and further, the stability of the viscosity of the fine fibrous cellulose dispersion liquid is excellent. In addition, as compared to that in the above-described method of adjusting the number of defibering steps, the time required for viscosity reduction is short, and thus it is possible to reduce the viscosity at a lower cost.

Detailed reasons of obtaining the above-described effects are unclear, but some are assumed as follows. In the present invention, the fine fibrous cellulose is treated with an enzyme, and is further treated with a protease, or a chlorine-containing oxo acid or a salt thereof, and thus the fine fibrous cellulose is enzymatically decomposed by the enzyme so that it is possible to reduce the viscosity of the fine fibrous cellulose dispersion liquid. However, if decomposition of the fine fibrous cellulose by the enzyme is excessively progressed, original functions possessed by the fine fibrous cellulose, such as a function possessed by the fine fibrous cellulose as a filler (reinforcing material) or a function as a thickener, are lost.

A heat treatment has conventionally been performed many times to inactivate the enzyme after the enzyme treatment, but when the heat treatment is performed, there is a concern that the fine fibrous cellulose may be colored.

In the present invention, the fine fibrous cellulose treated with the enzyme is further treated with the protease, or treated with the chlorine-containing oxo acid or a salt thereof so that the enzyme is inactivated and also a viscosity reduction is caused by the protease or the chlorine-containing oxo acid or a salt thereof itself. Thus, it is thought that the fine fibrous cellulose dispersion liquid excellent in the viscosity stability is obtained because a new viscosity reduction is realized, and also, the enzyme is inactivated.

When the fine fibrous cellulose-containing composition contains the chlorine-containing oxo acid or a salt thereof, it is thought that due to inclusion of the chlorine-containing oxo acid or a salt thereof, the molecular weight is reduced through cutting of a molecular chain in a part of the fine fibrous cellulose, and then the viscosity reduction of the dispersion liquid is caused. However, it may be thought that not only the viscosity reduction by the chlorine-containing oxo acid or a salt thereof is caused by the reduction of the molecular weight, but also the viscosity reduction of the dispersion liquid is caused because the chlorine-containing oxo acid or a salt thereof causes aggregation of the fine fibrous cellulose to the extent that optical properties such as haze or transmittance are not deteriorated.

In addition, even a small amount of protein contained in a cellulose raw material is involved in yellowing of the fine fibrous cellulose-containing composition, especially yellowing at the time of heating, and also, it is thought that even when a protein is mixed during storage due to some reasons, such a protein is involved in the yellowing at the time of heating. When the fine fibrous cellulose-containing composition contains the protease, there is an effect in that yellowing caused by heating is further suppressed because the protein causing the yellowing is decomposed by the protease. The protease to be added is a protein, but the addition amount is a small amount, and thus an influence on the yellowing of the fine fibrous cellulose-containing composition is slight.

Hereinafter, the present invention will be described in more detail.

<Fine Fibrous Cellulose>

The fine fibrous cellulose-containing composition of the present invention contains fine fibrous cellulose, and the corresponding fine fibrous cellulose is fibrous cellulose having a fiber width of 1,000 nm or less. The fiber width of the fibrous cellulose can be measured by, for example, observation with an electron microscope.

The fiber width of the fine fibrous cellulose is preferably 100 nm or less, more preferably 30 nm or less, further preferably 8 nm or less. In addition, the fiber width is preferably 2 nm or more.

The average fiber width of the fine fibrous cellulose is, for example, 1,000 nm or less. The average fiber width of the fine fibrous cellulose is preferably, for example, 2 nm or more and 1,000 nm or less, more preferably 2 nm or more and 100 nm or less, further preferably 2 nm or more and 50 nm or less, particularly preferably 2 nm or more and 10 nm or less. When the average fiber width of the fine fibrous cellulose is set to 2 nm or more, the dissolution as a cellulose molecule in water may be suppressed, so that the effect of improvement in the strength or rigidity, and the dimensional stability due to the fine fibrous cellulose can be more easily exhibited. The fine fibrous cellulose is, for example, single fibrous cellulose.

The average fiber width of the fibrous cellulose is measured by using, for example, an electron microscope in the following manner. First, an aqueous suspension of fibrous cellulose with a concentration of 0.05% by mass or more and 0.1% by mass or less is prepared, and the suspension is cast on a hydrophilized carbon film-coated grid to obtain a sample for TEM observation. When fibers with a wide width are included, an SEM image of the surface cast on glass may be observed. Then, observation is performed through an electron microscope image at any of magnifications of ×1,000, ×5,000, ×10,000 and ×50,000 according to the width of fibers to be observed. Meanwhile, the sample, the observation conditions, and the magnification are adjusted to satisfy the following conditions.

(1) one straight line X is drawn at an arbitrary position within an observation image, and 20 or more fibers intersect the corresponding straight line X.
(2) a straight line Y perpendicularly intersecting the corresponding straight line is drawn within the same image, and 20 or more fibers intersect the corresponding straight line Y.

In regard to the observation images satisfying the conditions, widths of fibers intersecting the straight lines X and the straight lines Y are visually read. In this manner, three or more sets of observation images on at least surface portions that do not overlap each other are obtained. Next, for each image, widths of fibers intersecting the straight line X and the straight line Y are read. Accordingly, at least 120 (=20×2×3) fiber widths are read. Then, the average value of the read fiber widths is set as an average fiber width of the fibrous cellulose.

The fiber length of the fine fibrous cellulose is not particularly limited, but is preferably, for example, 0.1 μm or more and 1,000 μm or less, more preferably 0.1 μm or more and 800 μm or less, further preferably 0.1 μm or more and 600 μm or less. When the fiber length is set within the range, destruction of a crystal region of the fine fibrous cellulose may be suppressed. In addition, it is also possible to set the slurry viscosity of the fine fibrous cellulose within an appropriate range. The fiber length of the fine fibrous cellulose may be obtained through, for example, image analysis by TEM, SEM, or AFM.

The degree of polymerization of the fine fibrous cellulose is preferably 430 or less, more preferably 400 or less, further preferably 350 or less from the viewpoint of reducing the viscosity of a fine fibrous cellulose dispersion liquid, and is preferably 200 or more, more preferably 250 or more, further preferably 300 or more from the viewpoint of functions as a reinforcing material, a thickener and the like.

The degree of polymerization of the fine fibrous cellulose is measured by a method described in Examples.

It is desirable that the fine fibrous cellulose has an I-type crystal structure. Here, the fact that the fine fibrous cellulose has the I-type crystal structure may be identified in a diffraction profile obtained from an wide-angle X-ray diffraction photograph using CuKa ($\lambda$=1.5418Å) monochromatized with graphite. Specifically, identification may be made from typical peaks present at two positions around $2\theta$=14° or more and 17° or less and around $2\theta$=22° or more and 23° or less.

The occupying ratio of the I-type crystal structure in the fine fibrous cellulose is preferably, for example, 30% or more, more preferably 40% or more, further preferably 50% or more. Accordingly, a more excellent performance may be expected in terms of a heat resistance and an occurrence of a low coefficient of linear thermal expansion. The degree of crystallinity may be obtained from a pattern in measurement of an X-ray diffraction profile, through a general method (Seagal et al, Textile Research Journal, vol 29, p 786, 1959).

The axial ratio (fiber length/fiber width) of the fine fibrous cellulose is not particularly limited, but is preferably, for example, 20 or more and 10,000 or less, more preferably 50 or more and 1,000 or less. By setting the axial ratio to the lower limit value or more, it is easy to form a sheet containing the fine fibrous cellulose. In addition, it is easy to obtain an appropriate viscosity thickening property when a solvent dispersion is produced. Setting the axial ratio to the upper limit value or less is preferable because, for example, handling such as dilution becomes easy when the fibrous cellulose is treated as an aqueous dispersion liquid.

The fine fibrous cellulose in the present embodiment has, for example, both a crystalline region and a non-crystalline region. In particular, the fine fibrous cellulose that has both a crystalline region and a non-crystalline region, and has a high axial ratio is realized by a fine fibrous cellulose production method to be described below.

The fine fibrous cellulose in the present embodiment has, for example, at least one type of an ionic substituent and a non-ionic substituent. From the viewpoint of improving the dispersibility of fibers within a dispersion medium, and increasing the defibration efficiency in a defibering process, it is more preferable that the fine fibrous cellulose has an ionic substituent. The ionic substituent may include, for example, either or both of an anionic group and a cationic group. In addition, the non-ionic substituent may include, for example, an alkyl group and an acyl group. In the present embodiment, it is particularly preferable to have the anionic group as the ionic substituent.

Here, the "ionic substituent" means an ionic group. Therefore, for example, when the fine fibrous cellulose has a carboxy group as the ionic group, even in a case where a hydroxy group included in the cellulose is oxidized to form the carboxy group, an ionic substituent is included.

A process of introducing an ionic substituent may not be performed on the fine fibrous cellulose.

The anionic group as the ionic substituent is preferably, for example, at least one type selected from a carboxymethyl group, a phosphoric acid group or a group derived from the phosphoric acid group (also simply referred to as a phosphoric acid group), a carboxy group or a group derived from the carboxy group (also simply referred to as a carboxy group), and a sulfonic acid group or a group derived from the sulfonic acid group (also simply referred to as a sulfone group), more preferably at least one type selected from a phosphoric acid group and a carboxy group, particularly preferably a phosphoric acid group.

The phosphoric acid group or the group derived from the phosphoric acid group is, for example, a substituent represented by the following formula (1), and is generalized as a phosphorus oxoacid group or a phosphorus oxoacid-

derived group.

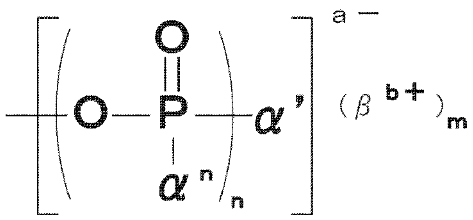

(1)

In the formula (1), a, b and n are natural numbers (in which a=b×m). a of $\alpha^1$, $\alpha^2$, ..., $\alpha^n$ and α' are $O^-$, and the rest are either R or OR. Each or all of $\alpha^n$ and α' may be $O^-$. Each R is a hydrogen atom, a saturated-linear hydrocarbon group, a saturated-branched hydrocarbon group, a saturated-cyclic hydrocarbon group, an unsaturated-linear hydrocarbon group, an unsaturated-branched hydrocarbon group, an unsaturated-cyclic hydrocarbon group, an aromatic group, or groups derived therefrom.

Examples of the saturated-linear hydrocarbon group include a methyl group, an ethyl group, an n-propyl group, and an n-butyl group, but are not particularly limited. Examples of the saturated-branched hydrocarbon group include an i-propyl group and a t-butyl group, but are not particularly limited. Examples of the saturated-cyclic hydrocarbon group include a cyclopentyl group and a cyclohexyl group, but are not particularly limited. Examples of the unsaturated-linear hydrocarbon group include a vinyl group and an allyl group, but are not particularly limited. Examples of the unsaturated-branched hydrocarbon group include an i-propenyl group and a 3-butenyl group, but are not particularly limited. Examples of the unsaturated-cyclic hydrocarbon group include a cyclopentenyl group and a cyclohexenyl group, but are not particularly limited. Examples of the aromatic group include a phenyl group and a naphthyl group, but are not particularly limited.

In addition, examples of the derived group in R include functional groups in which at least one type of functional groups such as a carboxy group, a hydroxy group, and an amino group is added or substituted in the main chain or the side chain of the various hydrocarbon groups, but are not particularly limited.

In addition, the number of carbon atoms constituting the main chain of R is not particularly limited, but is preferably 20 or less, more preferably 10 or less. When the number of carbon atoms constituting the main chain of R is set within the range, the molecular weight of the phosphoric acid group may be set within an appropriate range, so that penetration into a fiber raw material may be facilitated, and the yield of fine cellulose fibers may be increased.

$\beta^{b+}$ is a monovalent or higher cation composed of an organic substance or an inorganic substance. Examples of the monovalent or higher cation composed of the organic substance include aliphatic ammonium and aromatic ammonium, and examples of the monovalent or higher cation composed of the inorganic substance include ions of alkali metals such as sodium, potassium, or lithium, cations of divalent metals such as calcium or magnesium, and hydrogen ions, but are not particularly limited. For these, one type or a combination of two or more types may be applied. The monovalent or higher cation composed of the organic substance or the inorganic substance is preferably an ion of sodium or potassium which is hardly yellowed when the fiber raw material containing β is heated, and is easily industrially used, but is not particularly limited.

The phosphoric acid group is, for example, a divalent functional group corresponding to one obtained by removing a hydroxy group from phosphoric acid, and is specifically a group represented by $-PO_3H_2$. The group derived from the phosphoric acid group includes a substituent such as a salt of the phosphoric acid group, and a phosphoric acid ester group. The group derived from the phosphoric acid group may be contained in the fine fibrous cellulose, as a group in which the phosphoric acid group is condensed (for example, a pyrophosphoric acid group).

In addition, the phosphoric acid group may be, for example, a phosphorous acid group (a phosphonic acid group), and the substituent derived from the phosphoric acid group may be a salt of the phosphorous acid group, a phosphorous acid ester group, or the like.

The amount of ionic substituents introduced into the fine fibrous cellulose, that is, the content of ionic substituents in the fine fibrous cellulose is preferably, for example, 0.10 mmol/g or more per 1 g (mass) of the fine fibrous cellulose, more preferably 0.20 mmol/g or more, further preferably 0.60 mmol/g or more, particularly preferably 1.00 mmol/g or more. In addition, the amount of the ionic substituents introduced into the fine fibrous cellulose is preferably, for example, 5.20 mmol/g or less per 1 g (mass) of the fine fibrous cellulose, more preferably 3.65 mmol/g or less, further preferably 3.50 mmol/g or less, yet more preferably 3.00 mmol/g or less. When the introduction amount of the ionic substituents is set within the range, it is possible to facilitate the micronizing of the fiber raw material, and to increase the stability of the fine fibrous cellulose. In addition, when the introduction amount of the ionic substituents is set within the range, it is possible to exhibit good characteristics in various applications of the fine fibrous cellulose such as a thickener.

Here, the denominator in the unit mmol/g indicates the mass of the fibrous cellulose when the counterion of the ionic substituent is a hydrogen ion ($H^+$).

The amount of the ionic substituents introduced into the fine fibrous cellulose may be measured by, for example, a conductivity titration method. In the measurement using the conductivity titration method, while alkali such as a sodium hydroxide aqueous solution is added to the obtained fine fibrous cellulose-containing slurry, a change in the conductivity is determined so that the introduction amount is measured.

Fig. 1 is a graph illustrating the relationship between the electrical conductivity and the amount of NaOH added dropwise to fine fibrous cellulose having a phosphoric acid group.

For example, the amount of phosphoric acid groups introduced into the fine fibrous cellulose is measured as follows.

First, a fine fibrous cellulose-containing slurry is treated with a strong acid ion exchanged resin. As necessary, before the treatment with the strong acid ion exchanged resin, the same defibering process as a defibering step to be described below may be performed on a measurement target. Next, the change in the electrical conductivity is observed while a sodium hydroxide aqueous solution is added, so that a titration curve illustrated in Fig. 1 is obtained. As illustrated in Fig. 1, first, the electrical conductivity is sharply reduced (hereinafter, referred to as a "first region"). Then, the conductivity begins to slightly increase (hereinafter, referred to as a "second region"). Next, the increment of the conductivity increases (hereinafter, referred to as a "third region"). In addition, a boundary point between the second region and the third region is defined by a point at which a second differential value of the conductivity, that is, a change amount of the increment (gradient) of the conductivity, is maximized. In this manner, on the titration curve, three regions appear. Among them, the amount of required alkali for the first region is equal to the amount of strong acid groups in the slurry used for titration, and the amount of required alkali for the second region is equal to the amount of weak acid groups in the slurry used for titration. When the phosphoric acid group causes condensation, the weak acid groups are apparently lost, and the amount of required alkali for the second region is smaller than the amount of required alkali for the first region. Meanwhile, the amount of strong acid groups is equal to the amount of phosphorus atoms regardless of the presence/absence of condensation. Thus, simple description as the introduction amount of the phosphoric acid groups (or the amount of the phosphoric acid groups) or the introduction amount of the substituents (or the amount of the substituents) refers to the amount of the strong acid groups. Therefore, a value obtained by dividing the amount of required alkali (mmol) for the first region on the obtained titration curve by the solid content (g) in the slurry as a titration target becomes an introduction amount of the phosphoric acid groups (mmol/g).

The above-described introduction amount of the phosphoric acid groups (mmol/g) indicates the amount of the phosphoric acid groups included in acid-type fine fibrous cellulose (hereinafter, referred to as the amount of the phosphoric acid groups (acid type)) because the denominator indicates the mass of the acid-type fine fibrous cellulose. Meanwhile, when the counterion of the phosphoric acid group is replaced with an arbitrary cation C so as to have a charge equivalent, the denominator may be converted into the mass of fine fibrous cellulose in which the corresponding cation C is a counterion so that it is possible to obtain the amount of the phosphoric acid groups included in the fine fibrous cellulose in which the cation C is a counterion (hereinafter, the amount of the phosphoric acid groups (C type)).

That is, calculation is performed by the following calculation formula.

$$\text{Amount of phosphoric acid groups (C type)} = \text{amount of phosphoric acid groups (acid type)}/\{1+(W-1)\times A/1{,}000\}$$

A[mmol/g]: the total amount of anions derived from phosphoric acid groups included in fibrous cellulose (the sum of the amount of strong acid groups and the amount of weak acid groups in the phosphoric acid groups)

W: formula weight of cation C per valence (for example, Na is 23, and A1 is 9)

Fig. 2 is a graph illustrating the relationship between the electrical conductivity and the amount of NaOH added dropwise to fine fibrous cellulose having a carboxy group.

For example, the amount of carboxy groups introduced into the fine fibrous cellulose is measured as follows.

First, a fine fibrous cellulose-containing slurry is treated with a strong acid ion-exchanged resin. As necessary, before the treatment with the strong acid ion-exchanged resin, the same defibering process as a defibering step to be described below may be performed on a measurement target. Next, the change in the electrical conductivity is observed while a sodium hydroxide aqueous solution is added, so that a titration curve illustrated in Fig. 2 is obtained. As necessary, the same defibering process as the defibering step to be described below may be performed on the measurement target. As illustrated in Fig. 2, the titration curve is divided into a first region where the increment (gradient) of the conductivity becomes almost constant after the electrical conductivity is reduced, and thereafter, a second region where the increment (gradient) of the conductivity is increased. A boundary point between the first region and the second region is defined by a point at which a second differential value of the conductivity, that is, a change amount of the increment (gradient) of the conductivity is maximized. Then, a value obtained by dividing the amount of required alkali (mmol) for the first region on the titration curve by the solid content (g) in the fine fibrous cellulose-containing slurry as a titration target

becomes an introduction amount of the carboxy groups (mmol/g).

The above-described introduction amount of the carboxy groups (mmol/g) indicates the amount of the carboxy groups included in acid-type fine fibrous cellulose (hereinafter, referred to as the amount of the carboxy groups (acid type)) because the denominator is the mass of the acid-type fine fibrous cellulose. Meanwhile, when the counterion of the carboxy group is replaced with an arbitrary cation C so as to have a charge equivalent, the denominator may be converted into the mass of fine fibrous cellulose in which the corresponding cation C is a counterion so that it is possible to obtain the amount of the carboxy groups included in the fibrous cellulose in which the cation C is a counterion (hereinafter, the amount of the carboxy groups (C type)).

That is, calculation is performed by the following calculation formula.

$$\text{Amount of carboxy groups (C type)} = \text{amount of carboxy groups (acid type)}/\{1+(W-1)\times(\text{amount of carboxy groups (acid type)})/1{,}000\}$$

W: formula weight of cation C per valence (for example, Na is 23, and Al is 9)

<Method of Producing Fine Fibrous Cellulose>

(Cellulose-Containing Fiber Raw Material)

Fine fibrous cellulose is produced from a cellulose-containing fiber raw material (hereinafter, also simply referred to as a "fiber raw material").

The cellulose-containing fiber raw material is not particularly limited, but pulp is preferably used because it is easily available and inexpensive. Examples of the pulp include wood pulp, non-wood pulp, and deinked pulp. The wood pulp is not particularly limited, but examples thereof include chemical pulps such as broad leaved tree kraft pulp (LBKP), needle leaved tree kraft pulp (NBKP), sulfite pulp (SP), dissolving pulp (DP), soda pulp (AP), unbleached kraft pulp (UKP) and oxygen bleached kraft pulp (OKP), semi-chemical pulps such as semi-chemical pulp (SCP) and chemigroundwood pulp (CGP), and mechanical pulps such as ground wood pulp (GP) and thermo-mechanical pulp (TMP, BCTMP). The non-wood pulp is not particularly limited, but examples thereof include cotton-based pulps such as cotton linter and cotton lint, and non-wood-based pulps such as hemp, straw, bamboo, and bagasse. The deinked pulp is not particularly limited, but examples thereof include deinked pulp whose raw material is used paper. For the pulp in the present embodiment, one of the types may be used alone, or two or more types may be mixed and used.

Among the pulps, from the viewpoint of ease of availability, for example, wood pulp and deinked pulp are preferable. In addition, among the wood pulps, from the viewpoint of a high cellulose ratio and a high yield of fine fibrous cellulose during a defibering process, or from the viewpoint of obtaining fine fibrous cellulose of long fibers with a high axial ratio, in which decomposition of cellulose in pulp is low, for example, chemical pulp is more preferable, and kraft pulp and sulfite pulp are further preferable. When the fine fibrous cellulose of long fibers with a high axial ratio is used, the viscosity tends to be increased.

Examples of the cellulose-containing fiber raw material include cellulose contained in ascidians or bacteria cellulose produced by acetic acid bacteria.

In addition, instead of the cellulose-containing fiber raw material, in a part thereof, fibers formed by linear nitrogen-containing polysaccharide polymers such as chitin and chitosan may also be used.

In order to obtain the above-described fine fibrous cellulose in which an ionic substituent is introduced, it is desirable to include an ionic substituent introducing step of introducing the ionic substituent into the above-described cellulose-containing fiber raw material, a washing step, an alkali treatment step (neutralization step), and a defibering step in this order, and instead of the washing step, or in addition to the washing step, an acid treatment step may be included. The ionic substituent introducing step may be exemplified with a phosphoric acid group introducing step and a carboxy group introducing step. Hereinafter, each will be described.

(Ionic Substituent Introducing Step)

[Phosphoric Acid Group Introducing Step]

A step of introducing a phosphoric acid group (a phosphoric acid group introducing step) into cellulose fibers will be described below.

The phosphoric acid group introducing step is a step in which at least one type of compound (hereinafter, also referred to as a "compound A") selected from compounds capable of introducing a phosphoric acid group by reacting

with a hydroxy group included in the cellulose-containing fiber raw material is allowed to act on the cellulose-containing fiber raw material. Through this step, phosphoric acid group-introduced fibers are obtained.

In the phosphoric acid group introducing step according to the present embodiment, the reaction between the cellulose-containing fiber raw material and the compound A may be performed in the presence of at least one type (hereinafter, also referred to as a "compound B") selected from urea and its derivatives. Meanwhile, in a state where the compound B is not present, the reaction between the cellulose-containing fiber raw material and the compound A may be performed.

As an example of a method of allowing the compound A to act on the fiber raw material in the coexistence with the compound B, a method of mixing the compound A and the compound B with the fiber raw material in a dry state, a wet state or a slurry state may be exemplified. Among them, in terms of high uniformity of the reaction, it is preferable to use the fiber raw material in a dry state or a wet state, and it is particularly preferable to use the fiber raw material in a dry state. The form of the fiber raw material is not particularly limited, but is preferably, for example, cotton-like or thin sheet-like.

A method may be exemplified in which each of the compound A and the compound B, in a powder state or in a state of a solution obtained through dissolution in a solvent or in a melted state made through heating to a melting point or higher, is added to the fiber raw material. Among them, in terms of high uniformity of the reaction, addition in a state of a solution obtained through dissolution in a solvent, in particular, in an aqueous solution state, is preferable. In addition, the compound A and the compound B may be added to the fiber raw material at the same time, may be individually separately added, or may be added as a mixture. The method of adding the compound A and the compound B is not particularly limited, but when the compound A and the compound B are in a solution state, the fiber raw material may be immersed in the solution to absorb the liquid and then may be taken out, or the solution may be added dropwise to the fiber raw material. In addition, required amounts of the compound A and the compound B may be added to the fiber raw material, or after excess amounts of the compound A and the compound B are separately added to the fiber raw material, the excess compound A and the compound B may be removed through pressing or filtering.

The compound A used in the present embodiment may be exemplified with a compound that has a phosphorus atom and is capable of forming an ester bond with cellulose, and specific examples thereof include phosphoric acid or a salt thereof, phosphorous acid or a salt thereof, dehydrated condensed phosphoric acid or a salt thereof, and phosphoric anhydride (diphosphorus pentoxide), but are not particularly limited. As for the phosphoric acid, those having various purities may be used, and, examples thereof include 100% phosphoric acid (orthophosphoric acid) or 85% phosphoric acid. Examples of the phosphorous acid include 99% phosphorous acid (phosphonic acid). The dehydrated condensed phosphoric acid is obtained by condensing two or more molecules of the phosphoric acid through a dehydration reaction, and examples thereof include pyrophosphoric acid, and polyphosphoric acid. Examples of phosphate, phosphite, and dehydrated condensed phosphate include lithium salts, sodium salts, potassium salts, and ammonium salts of phosphoric acid, phosphorous acid or dehydrated condensed phosphoric acid, and these may have various degrees of neutralization.

Among them, from the viewpoint of easier improvement in the defibration efficiency in a defibering step to be described below, a low cost and an ease of industrial application due to high introduction efficiency of the phosphoric acid group, phosphoric acid, a sodium salt of phosphoric acid, a potassium salt of phosphoric acid, or an ammonium salt of phosphoric acid is preferable, and phosphoric acid, sodium dihydrogen phosphate, disodium hydrogen phosphate, or ammonium dihydrogen phosphate is more preferable.

The amount of the compound A added to the fiber raw material is not particularly limited, but, for example, in a case where the addition amount of the compound A is converted into a phosphorus atomic weight, the amount of phosphorus atoms added to the fiber raw material (absolute dry mass, hereinafter also referred to as "absolute dry mass") is preferably 0.5% by mass or more and 100% by mass or less, more preferably 1% by mass or more and 50% by mass or less, further preferably 2% by mass or more and 30% by mass or less. When the amount of phosphorus atoms added to the fiber raw material is set within the range, the yield of the fine fibrous cellulose can be further improved. Meanwhile, when the amount of phosphorus atoms added to the fiber raw material is set to the upper limit value or less, a yield improving effect and a cost can be balanced. Here, the "absolute dry mass (absolute dry mass)" refers to the mass in an absolute dry state where a constant amount is reached, which is measured by the method described in ISO 638:2008.

As described above, the compound B used in the present embodiment is at least one type selected from urea and its derivatives. Examples of the compound B include urea, biuret, 1-phenylurea, 1-benzylurea, 1-methylurea, and 1-ethylurea.

From the viewpoint of improving the uniformity of a reaction, it is preferable to use the compound B as an aqueous solution. In addition, from the viewpoint of further improving the uniformity of a reaction, it is preferable to use an aqueous solution in which both the compound A and the compound B are dissolved.

The amount of the compound B added to the fiber raw material (absolute dry mass) is not particularly limited, but is preferably, for example, 1% by mass to 500% by mass, more preferably 10% by mass or more and 400% by mass or less, further preferably 100% by mass or more and to 350% by mass or less.

In the reaction between the cellulose-containing fiber raw material and the compound A, in addition to the compound B, for example, amides or amines may be included in the reaction system. Examples of the amides include formamide, dimethylformamide, acetamide, and dimethylacetamide. Examples of the amines include methylamine, ethylamine, trimethylamine, triethylamine, monoethanolamine, diethanolamine, triethanolamine, pyridine, ethylenediamine, and hexamethylenediamine. Among them, especially, triethylamine is known to act as a good reaction catalyst.

In the phosphoric acid group introducing step, it is desirable that after the compound A, etc. are added or mixed to/with the fiber raw material, a heat treatment is carried out on the corresponding fiber raw material. As for the heat treatment temperature, it is desirable to choose a temperature at which a thermal decomposition or a hydrolysis reaction of fibers can be suppressed while the phosphoric acid group can be efficiently introduced. The heat treatment temperature is preferably, for example, 50°C or more and 300°C or less, more preferably 100°C or more and 250°C or less, further preferably 130°C or more and 200°C or less. In addition, for the heat treatment, devices having various heat media may be used, and examples thereof include a stirring drying device, a rotary drying device, a disk drying device, a roll-type heating device, a plate-type heating device, a fluidized bed drying device, an air flow drying device, a vacuum-drying device, an infrared heating device, a far-infrared heating device, or a microwave heating device.

In the heat treatment according to the present embodiment, it is possible to employ, for example, a method of performing heating after adding the compound A to a thin sheet-like fiber raw material through a method such as impregnation, or a method of performing heating while kneading or stirring the fiber raw material and the compound A by a kneader or the like. This makes it possible to suppress an unevenness of the concentration of the compound A in the fiber raw material, and to more uniformly introduce the phosphoric acid groups onto the surfaces of cellulose fibers included in the fiber raw material. It may be thought that this is because as the drying is performed, when water molecules move to the surface of the fiber raw material, the dissolved compound A can be suppressed from being attracted to the water molecules by surface tension, and similarly moving to the surface of the fiber raw material (that is, unevenness of the concentration of the compound A is caused).

In addition, it is desirable that the heating device used for the heat treatment is, for example, a device capable of always discharging the water content retained by the slurry, and the water content produced according to a dehydration condensation (phosphate esterification) reaction between the compound A and a hydroxy group, etc. included in cellulose, etc. in the fiber raw material, to the outside of a device system. Examples of such a heating device include a blowing-type oven. By always discharging the water content within the device system, not only a hydrolysis reaction of a phosphoric acid ester bond, as a reverse reaction of phosphate esterification, can be suppressed, but also acid hydrolysis of a sugar chain within fibers can be suppressed. This makes it possible to obtain fine fibrous cellulose with a high axial ratio.

The time for the heat treatment is preferably, for example, 1 sec or more and 300 min or less after the water content is substantially removed from the fiber raw material, more preferably 1 sec or more and 1,000 sec or less, further preferably 10 sec or more and 800 sec or less. In the present embodiment, when the heating temperature and the heating time are set within appropriate ranges, the introduction amount of the phosphoric acid groups may be set within a preferable range.

The phosphoric acid group introducing step only has to be performed at least once, but may be repeatedly performed twice or more. By performing the phosphoric acid group introducing step twice or more, it is possible to introduce many phosphoric acid groups into the fiber raw material. In the present embodiment, as an example of a preferred embodiment, there may be a case where the phosphoric acid group introducing step is performed twice.

The amount of the phosphoric acid groups introduced into the fiber raw material is preferably, for example, 0.10 mmol/g or more per 1 g (mass) of the fine fibrous cellulose, more preferably 0.20 mmol/g or more, further preferably 0.60 mmol/g or more, particularly preferably 1.00 mmol/g or more. In addition, the amount of the phosphoric acid groups introduced into the fiber raw material is preferably, for example, 5.20 mmol/g or less per 1 g (mass) of the fine fibrous cellulose, more preferably 3.65 mmol/g or less, further preferably 3.00 mmol/g or less. When the introduction amount of the phosphoric acid groups is set within the range, it is possible to facilitate the micronizing of the fiber raw material and to increase the stability of the fine fibrous cellulose.

[Carboxy Group Introducing Step]

The carboxy group introducing step is performed by subjecting the cellulose-containing fiber raw material to oxidation treatment such as ozone oxidation, oxidation using a Fenton method, or a TEMPO oxidation treatment, or treatment with a compound having a carboxylic acid-derived group or its derivative, or an acid anhydride of a compound having a carboxylic acid-derived group or its derivative.

The compound having the carboxylic acid-derived group is not particularly limited, but examples thereof include dicarboxylic acid compounds such as maleic acid, succinic acid, phthalic acid, fumaric acid, glutaric acid, adipic acid, and itaconic acid, or tricarboxylic acid compounds such as citric acid, and aconitic acid. In addition, the derivative of the compound having the carboxylic acid-derived group is not particularly limited, but examples thereof include imidization products of an acid anhydride of a compound having a carboxy group, and derivatives of an acid anhydride of a compound

having a carboxy group. The imidization product of the acid anhydride of the compound having the carboxy group is not particularly limited, but examples thereof include imidization products of dicarboxylic acid compounds, such as maleimide, succinimide, and phthalimide.

The acid anhydride of the compound having the carboxylic acid-derived group is not particularly limited, but examples thereof include acid anhydrides of dicarboxylic acid compounds, such as maleic anhydride, succinic anhydride, phthalic anhydride, glutaric anhydride, adipic anhydride, and itaconic anhydride. In addition, the derivative of the acid anhydride of the compound having the carboxylic acid-derived group is not particularly limited, but examples thereof include those in which at least a part of hydrogen atoms of the acid anhydride of the compound having the carboxy group, such as dimethylmaleic anhydride, diethylmaleic anhydride, or diphenylmaleic anhydride, is substituted with a substituent such as an alkyl group or a phenyl group.

In the carboxy group introducing step, when the TEMPO oxidation treatment is performed, for example, it is desirable to perform the treatment under conditions where pH is 6 or more and to 8 or less. Such a treatment is also called a neutral TEMPO oxidation treatment. The neutral TEMPO oxidation treatment may be performed by adding, for example, pulp as the fiber raw material, a nitroxy radical such as TEMPO (2,2,6,6-tetramethylpiperidine-1-oxyl) as a catalyst, and sodium hypochlorite as a sacrificial reagent, to sodium phosphate buffer (pH=6.8). also, through coexistence with sodium chlorite, aldehyde generated in the oxidation process can be efficiently oxidized to a carboxy group.

In addition, in the TEMPO oxidation treatment, the treatment may be performed under conditions where pH is 10 or more and to 11 or less. Such a treatment is also called an alkali TEMPO oxidation treatment. The alkali TEMPO oxidation treatment may be performed by adding, for example, a nitroxy radical such as TEMPO as a catalyst, sodium bromide as a co-catalyst, and sodium hypochlorite as an oxidant, to pulp as the fiber raw material.

The amount of carboxy groups introduced into the fiber raw material changes depending on the type of the substituent, and is preferably 0.10 mmol/g or more per 1 g (mass) of the fine fibrous cellulose, more preferably 0.20 mmol/g or more, further preferably 0.60 mmol/g or more, particularly preferably 0.90 mmol/g or more, for example, in a case where the carboxy groups are introduced through TEMPO oxidation. In addition, it is preferably 2.5 mmol/g or less, more preferably 2.20 mmol/g or less, further preferably 2.00 mmol/g or less. Otherwise, when the substituent is a carboxymethyl group, it may be 5.8 mmol/g or less per 1 g (mass) of the fine fibrous cellulose.

(Washing Step)

In the method of producing the fine fibrous cellulose in the present embodiment, as necessary, a washing step may be performed on fibers to which the substituent such as an ionic substituent is introduced. The washing step is performed by washing the substituent-introduced fibers with, for example, water or an organic solvent. In addition, the washing step may be performed after each step to be described below, and the number of times washing is carried out in each washing step is not particularly limited.

(Alkali Treatment Step)

When the fine fibrous cellulose is produced, an alkali treatment may be performed on the fiber raw material between the step of introducing the substituent such as the ionic substituent and a defibering step to be described below. The method for the alkali treatment is not particularly limited, but examples thereof include a method of immersing the substituent-introduced fibers in an alkali solution.

An alkali compound contained in the alkali solution is not particularly limited, and may be an inorganic alkali compound or may be an organic alkali compound. In the present embodiment, it is desirable to use, for example, sodium hydroxide or potassium hydroxide as the alkali compound in terms of high versatility. In addition, a solvent contained in the alkali solution may be either water or an organic solvent. Among them, the solvent contained in the alkali solution is preferably a polar solvent, including water, or a polar organic solvent (for example, alcohol), more preferably an aqueous solvent including at least water. As the alkali solution, in terms of high versatility, for example, a sodium hydroxide aqueous solution, or a potassium hydroxide aqueous solution is preferable.

The temperature of the alkali solution in the alkali treatment step is not particularly limited, but is preferably, for example, 5°C or more and 80°C or less, more preferably 10°C or more and 60°C or less. The immersion time of the substituent-introduced fibers in the alkali solution in the alkali treatment step is not particularly limited, but is preferably, for example, 5 min or more and 30 min or less, more preferably 10 min or more and 20 min or less. The use amount of the alkali solution in the alkali treatment is not particularly limited, but is preferably, for example, 100% by mass or more and 100,000% by mass or less, more preferably 1,000% by mass or more and 10,000% by mass or less with respect to the absolute dry mass of the substituent-introduced fibers.

In order to reduce the use amount of the alkali solution in the alkali treatment step, the substituent-introduced fibers may be washed with water or an organic solvent after the step of introducing the substituent such as the ionic substituent before the alkali treatment step. After the alkali treatment step, before the defibering step, from the viewpoint

of improving handleability, it is desirable that the substituent-introduced fibers which have been subjected to the alkali treatment are washed with water or an organic solvent.

(Acid Treatment Step)

When the fine fibrous cellulose is produced, an acid treatment may be performed on the fiber raw material between the step of introducing the substituent such as the ionic substituent, and the defibering step to be described below. For example, the substituent introducing step, the acid treatment, the alkali treatment and the defibering process may be performed in this order.

The method for the acid treatment is not particularly limited, but examples thereof include a method of immersing the fiber raw material in an acid-containing acidic liquid. The concentration of the acidic liquid to be used is not particularly limited, but is preferably, for example, 10% by mass or less, more preferably 5% by mass or less. In addition, pH of the acidic liquid to be used is not particularly limited, but is preferably, for example, 0 or more and 4 or less, more preferably 1 or more and 3 or less. Examples of the acid contained in the acidic liquid include inorganic acid, sulfonic acid, and carboxylic acid. Examples of the inorganic acid include sulfuric acid, nitric acid, hydrochloric acid, hydrobromic acid, hydroiodic acid, hypochlorous acid, chlorous acid, chloric acid, perchloric acid, phosphoric acid, and boric acid. Examples of the sulfonic acid include methanesulfonic acid, ethanesulfonic acid, benzenesulfonic acid, p-toluenesulfonic acid, and trifluoromethanesulfonic acid. Examples of the carboxylic acid include formic acid, acetic acid, citric acid, gluconic acid, lactic acid, oxalic acid, and tartaric acid. Among them, it is particularly preferable to use hydrochloric acid or sulfuric acid.

The temperature of the acid solution in the acid treatment is not particularly limited, but is preferably, for example, 5°C or more and 100°C or less, more preferably 20°C or more and 90°C or less. The time of immersion in the acid solution in the acid treatment is not particularly limited, but is preferably, for example, 5 min or more and 120 min or less, more preferably 10 min or more and 60 min or less. The use amount of the acid solution in the acid treatment is not particularly limited, but is preferably, for example, 100% by mass or more and 100,000% by mass or less, more preferably 1,000% by mass or more and 10,000% by mass or less with respect to the absolute dry mass of the fiber raw material.

(Defibering Process)

The fine fibrous cellulose is obtained by defibering the fibers into which the substituent such as the ionic substituent is introduced in the defibering step.

In the defibering step, for example, a defibering device may be used. The defibering device is not particularly limited, but, examples thereof include a high-speed defibrator, a grinder (a millstone-type crusher), a high-pressure homogenizer or an ultra-high-pressure homogenizer, a high-pressure collision-type crusher, a ball mill, a bead mill, a disc-type refiner, a conical refiner, a twin-screw kneader, a vibration mill, a homomixer under high-speed rotation, an ultrasonic disperser, and a beater. Among the defibering devices, it is more preferable to use a high-speed defibrator, a high-pressure homogenizer, or an ultra-high-pressure homogenizer which is less affected by a crushing medium and has a low risk of contamination.

In the defibering step, for example, it is preferable that the substituent-introduced fibers are diluted with a dispersion medium and then take a slurry form. As for the dispersion medium, one type or two or more types selected from water, and organic solvents such as a polar organic solvent may be used. The polar organic solvent is not particularly limited, but, for example, alcohols, polyhydric alcohols, ketones, ethers, esters, an aprotic polar solvent and the like are preferable. Examples of the alcohols include methanol, ethanol, isopropanol, n-butanol, and isobutylalcohol. Examples of the polyhydric alcohols include ethyleneglycol, propyleneglycol, and glycerin. Examples of the ketones include acetone and methylethylketone (MEK). Examples of the ethers include diethylether, tetrahydrofuran, ethyleneglycolmonomethylether, ethyleneglycolmonoethylether, ethyleneglycolmono n-butylether, and propyleneglycolmonomethylether. Examples of the esters include ethyl acetate, and butyl acetate. Examples of the aprotic polar solvent include dimethylsulfoxide (DMSO), dimethylformamide (DMF), dimethylacetamide (DMAc), and N-methyl-2-pyrrolidinone (NMP).

The solid content concentration of the fibrous cellulose at the time of the defibering process may be properly set. In addition, the slurry obtained by dispersing the substituent-introduced fibers in the dispersion medium may contain, for example, any solid content other than the substituent-introduced fibers, such as urea having a hydrogen bondability.

<Enzyme-Derived Protein>

The fine fibrous cellulose-containing composition of the present invention contains an enzyme-derived protein. The reason the fine fibrous cellulose-containing composition of the present invention contains the enzyme-derived protein is because the fine fibrous cellulose dispersion liquid is treated with an enzyme and is further treated with a protease to be described below, or a chlorine-containing oxo acid or a salt thereof so that the enzyme is denatured and inactivated and then becomes the enzyme-derived protein. That is, the "enzyme-derived protein" is a protein whose enzyme activity

is lost because the enzyme is enzymatically decomposed or denatured by at least the protease or the chlorine-containing oxo acid or a salt thereof and then is inactivated.

[Enzyme]

The enzyme used in the present invention is not particularly limited, but is preferably a cellulose-degrading enzyme, that is, a cellulase, from the viewpoint of reducing the molecular weight of the cellulose and reducing the viscosity of the dispersion liquid.
The cellulase is an enzyme that mainly produces cellobiose by hydrolyzing a $\beta 1 \rightarrow 4$ glucosidic bond in the cellulose. As the cellulase, there are endoglucanase (EC 3.2.1.4) that randomly decomposes cellulose chains and cellobiohydrolase (also referred to as exoglucanase, EC 3.2.1.91) that produces cellobiose through decomposition from the reducing terminal of cellulose.
Among them, the enzyme to be used in the present invention preferably contains at least endoglucanase, and may contain both endoglucanase and cellobiohydrolase, but it is more preferably only endoglucanase.
The addition amount of the enzyme (preferably cellulase, more preferably endoglucanase) is, based on 1 mg of the fine fibrous cellulose, preferably 0.0001 U or more, more preferably 0.0005 U or more, further preferably 0.001 U or more from the viewpoint of reducing the molecular weight of the fine fibrous cellulose, and preferably 1.0 U or less, more preferably 0.1 U or less, further preferably 0.01 U or less from the viewpoint of the economy and the suppression of excessive reduction of the molecular weight.
U is an international unit of an enzyme, and is defined by the amount of enzyme activity by which 1 μmol of substrate is converted into a product for 1 min.

<Protease>

The first fine fibrous cellulose-containing composition of the present invention contains a protease. Apart of the protease may be inactivated by heat, light, an acid, a base or the like.
The protease to be used in the present invention is not particularly limited, and may be exopeptidase that cleaves one to two amino residues at a time from the end of the protein or peptide chain sequence or may be endopeptidase that cleaves the center of the protein or peptide chain sequence. However, endopeptidase is preferable from the viewpoint of efficiently inactivating an enzyme.
The optimal pH of the protease to be used in the present invention is preferably 3 or more and 12 or less, more preferably 4 or more and 11 or less, further preferably 5 or more and 10 or less in terms of the ease of treatment by the protease.
In addition, the optimal temperature of the protease to be used in the present invention is preferably 10°C or more and 80°C or less, more preferably 15°C or more and 70°C or less, further preferably 20°C or more and 60°C or less from the viewpoint of the ease of protease treatment, and the suppression of heating-induced deterioration of the fine fibrous cellulose-containing composition.
In the first fine fibrous cellulose-containing composition of the present invention, the content of the protease is preferably 0.0002 U or more, more preferably 0.001 U or more, further preferably 0.005 U or more based on 1 mg of the fine fibrous cellulose, and is preferably 20 U or less, more preferably 2.0 U or less, further preferably 1.0 U or less from the viewpoint of the economy and the suppression of excessive addition of the protease as a protein.
In the first fine fibrous cellulose-containing composition of the present invention, the content of the protease based on 1 mg of an enzyme-derived protein is preferably 1 U or more, more preferably 10 U or more, further preferably 100 U or more from the viewpoint of inactivating an enzyme, and is preferably 1,000,000 U or less, more preferably 100,000 U or less, further preferably 30,000 U or less from the viewpoint of the economy and the suppression of excessive addition of the protease as a protein.
The U is an international unit of an enzyme, and is defined by enzyme activity by which 1 μmol of substrate is converted into a product for 1 min.

<Chlorine-Containing Oxo Acid or Salt Thereof>

The second fine fibrous cellulose-containing composition of the present invention contains a chlorine-containing oxo acid or a salt thereof.
Examples of the chlorine-containing oxo acid include hypochlorous acid, chlorous acid, chloric acid, and perchloric acid, and from the viewpoint of the ease of availability, the stability, and the like, hypochlorous acid is preferable.
In addition, examples of the salt of the chlorine-containing oxo acid include salts of hypochlorous acid such as sodium hypochlorite, potassium hypochlorite, and calcium hypochlorite; salts of chlorous acid such as sodium chlorite, potassium chlorite, copper chlorite, and barium chlorite; salts of chloric acid such as sodium chlorate, potassium chlorate,

ammonium chlorate, calcium chlorate, barium chlorate, zinc chlorate, and silver chlorate; and salts of perchloric acid such as ammonium perchlorate, potassium perchlorate, sodium perchlorate, calcium perchlorate, silver chlorite, and magnesium perchlorate.

Among them, as the chlorine-containing oxo acid or a salt thereof, it is preferable to contain a salt of chlorine-containing oxo acid, it is more preferable to contain a salt of hypochlorous acid, it is further preferable to contain at least one selected from the group consisting of sodium hypochlorite, potassium hypochlorite, and calcium hypochlorite, it is particularly preferable to contain at least one selected from the group consisting of sodium hypochlorite and potassium hypochlorite, and it is most preferable to contain sodium hypochlorite.

In the second fine fibrous cellulose-containing composition of the present invention, the content of the chlorine-containing oxo acid or a salt thereof based on 100 parts by mass of the fine fibrous cellulose is preferably 0.0001 parts by mass or more, more preferably 0.0003 parts by mass or more, further preferably 0.001 parts by mass or more, yet more preferably 0.01 parts by mass or more, still more preferably 0.1 parts by mass or more from the viewpoint of inactivating an enzyme, and also obtaining a sufficient viscosity reducing effect, and is preferably 10 parts by mass or less, more preferably 5 parts by mass or less, further preferably 3 parts by mass or less, still more preferably 1 part by mass or less from the viewpoint of suppressing yellowing of the fine fibrous cellulose.

When the chlorine-containing oxo acid or a salt thereof is used as an oxidant in the oxidation treatment of the cellulose raw material, and then a washing step is performed as in the case where a defibering process is performed after a washing step is performed, the content of the chlorine-containing oxo acid or a salt thereof becomes less than 0.0001 parts by mass based on 100 parts by mass of the fine fibrous cellulose.

In the second fine fibrous cellulose-containing composition of the present invention, the content of the chlorine-containing oxo acid or a salt thereof based on 100 parts by mass of an enzyme-derived protein is preferably 10 parts by mass or more, more preferably 100 parts by mass or more, further preferably 1,000 parts by mass or more from the viewpoint of inactivating an enzyme, and is preferably 1,000,000 parts by mass or less, more preferably 100,000 parts by mass or less, further preferably 10,000 parts by mass or less from the viewpoint of appropriately reducing the molecular weight.

<Fine Fibrous Cellulose-containing composition>

In the present invention, the fine fibrous cellulose-containing composition contains the fine fibrous cellulose, the enzyme-derived protein, and the protease or the chlorine-containing oxo acid or a salt thereof. Further, besides these, other components may be contained. Examples of other components include a water-soluble polymer, and a surfactant.

Examples of the water-soluble polymer include synthetic water-soluble polymers such as a carboxyvinyl polymer, polyvinylalcohol, an alkyl methacrylate-acrylic acid copolymer, polyvinylpyrrolidone, sodium polyacrylate, polyethyleneglycol, diethyleneglycol, triethyleneglycol, polyethyleneoxide, propyleneglycol, dipropyleneglycol, polypropyleneglycol, isopreneglycol, hexyleneglycol, 1,3-butyleneglycol, and polyacrylamide; thickening polysaccharides such as xanthan gum, guar gum, tamarind gum, carrageenan, locust bean gum, quince seed, alginic acid, pullulan, carrageenan, and pectin; cellulose derivatives such as carboxymethylcellulose, methylcellulose, and hydroxyethylcellulose; starches such as cationized starch, raw starch, oxidized starch, etherified starch, esterified starch, and amylose; glycerins such as glycerin, diglycerin, and polyglycerin; hyaluronic acid, and a metal salt of the hyaluronic acid.

The content of the water-soluble polymer in the fine fibrous cellulose-containing composition is preferably, for example, 0.05 parts by mass or more, more preferably 0.1 parts by mass or more, further preferably 0.5 parts by mass or more based on 100 parts by mass of the fine fibrous cellulose. Meanwhile, the content of the water-soluble polymer in the fine fibrous cellulose-containing composition is preferably, for example, 500 parts by mass or less, more preferably 200 parts by mass or less, further preferably 100 parts by mass or less based on 100 parts by mass of the fine fibrous cellulose. When the content of the water-soluble polymer is set within the described range, it is possible to exhibit both properties of the fine fibrous cellulose and properties of the water-soluble polymer.

Examples of the surfactant include a nonionic surfactant, an anionic surfactant, a cationic surfactant, and an amphoteric surfactant. Examples of the nonionic surfactant include polyoxyalkylene alkyl ether, polyglycerin fatty acid ester, glycerin mono fatty acid ester, glycerin di-fatty acid ester, pluronic, sorbitan fatty acid ester, polyoxyethylene sorbitan fatty acid ester, sucrose fatty acid ester, polyoxyethylene acyl ester, alkyl polyglycoside, fatty acid methyl glycoside ester, alkyl methyl glucamide, and fatty acid alkanolamide. Examples of the anionic surfactant include sodium oleate, potassium oleate, sodium laurate, sodium dodecylbenzenesulfonate, sodium alkylnaphthalenesulfonate, sodium dialkyl sulfosuccinate, sodium polyoxyethylene alkylether sulfate, sodium polyoxyethylene alkylallylether sulfate, sodium polyoxyethylene dialkyl sulfate, polyoxyethylene alkylether phosphoric acid ester, and polyoxyethylene alkylallylether phosphoric acid ester. Examples of the cationic surfactant include: quaternary ammonium salts such as an alkyltrimethylammonium salt, a dialkyldimethylammonium salt, an alkyldimethylbenzylammonium salt, an acylaminoethyldiethylammonium salt, an acylaminoethyldiethylamine salt, an alkylamidepropyldimethylbenzylammonium salt, an alkylpyridinium

salt, an alkylpyridinium sulfate, a stearamide methylpyridinium salt, an alkylquinolinium salt, an alkylisoquinolinium salt, fatty acidpolyethylenepolyamide, an acylaminoethylpyridinium salt, and an acylcolamino formylmethylpyridinium salt; salts of ester-bonded amines or ether-bonded quaternary ammoniums such as a stearoxy methylpyridinium salt, fatty acid triethanolamine, a fatty acid triethanolamine formic acid salt, trioxyethylene fatty acid triethanolamine, a cetyloxy methylpyridinium salt, and a p-isooctyl phenoxyethoxy ethyldimethylbenzylammonium salt; heterocyclic amines such as alkylimidazoline, 1-hydroxyethyl-2-alkylimidazoline, 1-acetylaminoethyl-2-alkylimidazoline, and 2-alkyl-4-methyl-4-hydroxymethyloxazoline; and amine derivatives such as polyoxyethylene alkylamine, N-alkyl propylene diamine, N-alkyl polyethylene polyamine, N-alkyl polyethylene polyamine dimethyl sulfate, alkyl biguanide, and long-chain amine oxide. Examples of the amphoteric surfactant include lauryldimethylaminoacetic acid betaine, cocamidopropyl betaine, lauramidopropyl betaine, and sodium cocoamphoacetate.

The content of the surfactant in the fine fibrous cellulose-containing composition is preferably, for example, 0.01 parts by mass or more, more preferably 0.05 parts by mass or more, further preferably 0.1 parts by mass or more based on 100 parts by mass of the fine fibrous cellulose. Meanwhile, the content of the surfactant in the fine fibrous cellulose-containing composition is preferably, for example, 5 parts by mass or less, more preferably 3 parts by mass or less, further preferably 2 parts by mass or less based on 100 parts by mass of the fine fibrous cellulose. When the content of the surfactant is set within the above-described range, it is possible to exhibit both properties of the fine fibrous cellulose, and properties of the surfactant.

Besides the water-soluble polymer and the surfactant, the fine fibrous cellulose-containing composition may contain, for example, one type or two or more types selected from an organic ion, a coupling agent, an inorganic layered compound, an inorganic compound, a levelling agent, a preservative, a defoamer, organic particles, a lubricant, an antistatic agent, a UV protecting agent, a dye, a pigment, a stabilizer, magnetic powder, an orientation accelerator, a plasticizer, a dispersant, and a cross-linking agent.

The fibrous cellulose-containing composition may contain a solvent. The solvent may include, for example, one or both of water and an organic solvent. Examples of the organic solvent include alcohols, polyhydric alcohols, ketones, ethers, esters, hydrocarbons, halogens, and an aprotic polar solvent. Examples of the alcohols include methanol, ethanol, isopropanol, n-butanol, and isobutylalcohol. Examples of the polyhydric alcohols include ethyleneglycol, propyleneglycol, and glycerin. Examples of the ketones include acetone, and methylethylketone (MEK). Examples of the ethers include diethylether, tetrahydrofuran, ethyleneglycolmonomethylether, ethyleneglycolmonoethylether, ethyleneglycolmono n-butylether, and propyleneglycolmonomethylether. Examples of the esters include ethyl acetate, and butyl acetate. Examples of the hydrocarbons include n-hexane, toluene, and xylene. Examples of the halogens include methylene chloride, trichloroethylene, and chloroform. Examples of the aprotic polar solvent include dimethylsulfoxide (DMSO), dimethylformamide (DMF), dimethylacetamide (DMAc), and N-methyl-2-pyrrolidinone (NMP).

The haze of a fine fibrous cellulose-containing composition slurry whose solid content concentration of the fine fibrous cellulose is adjusted to 0.2% by mass, which is measured at 23°C by using a glass cell with an optical path length of 1 cm in accordance with JIS K 7136, is preferably 2.0% or less. In addition, the haze is more preferably 1.5% or less, further preferably 1.0% or less. Meanwhile, the lower limit value of the haze is not particularly limited, and may be, for example, 0%. When the haze of the slurry of the fine fibrous cellulose-containing composition is set within this range, it is possible to form a sheet or the like with a higher transparency.

In the present embodiment, measurement may be performed by using, for example, a hazemeter (manufactured by MURAKAMI COLOR RESEARCH LABORATORY, Co., Ltd, HM-150). In addition, examples of the glass cell with an optical path length of 1 cm include a glass cell for a liquid with an optical path length of 1 cm (manufactured by FUJIWARA SCIENTIFIC Co., Ltd., MG-40, inverse optical path). Zero point measurement is performed with ion-exchanged water put in the same glass cell.

It is desirable that the solvent at the time of the haze measurement contains at least water, and the occupying content of water in the whole solvent is preferably 50% by mass or more, more preferably 70% by mass or more, further preferably 90% by mass or more, particularly preferably 95% by mass or more, and may be 100% by mass.

When the fine fibrous cellulose-containing composition is used for purposes in which the transparency is not required, the haze may be, for example 30% or more, may be 60% or more, or may be 90% or more.

In the present invention, the total light transmittance of the fine fibrous cellulose-containing composition slurry whose solid content concentration of the fine fibrous cellulose is adjusted to 0.2% by mass, which is measured by using a glass cell with an optical path length of 1 cm in accordance with JIS K 7361, is preferably 90% or more. In addition, the total light transmittance is more preferably 93% or more, further preferably 95% or more. Meanwhile, the upper limit value of the total light transmittance is not particularly limited, and may be, for example, 100%. When the total light transmittance of the slurry of the fine fibrous cellulose-containing composition is set within this range, it is possible to form a sheet or the like with a higher transparency.

In the present embodiment, the total light transmittance may be measured by using, for example, a hazemeter (manufactured by MURAKAMI COLOR RESEARCH LABORATORY Co., Ltd., HM-150). In addition, examples of the glass cell with an optical path length of 1 cm include a glass cell for a liquid with an optical path length of 1 cm (manufactured

by FUJIWARA SCIENTIFIC Co., Ltd., MG-40, inverse optical path). Zero point measurement is performed with ion-exchanged water put in the same glass cell.

It is desirable that the solvent at the time of the total light transmittance measurement contains at least water, and the occupying content of water in the whole solvent is preferably 50% by mass or more, more preferably 70% by mass or more, further preferably 90% by mass or more, particularly preferably 95% by mass or more, and may be 100% by mass.

When the fine fibrous cellulose-containing composition is used for purposes in which the transparency is not required, the transmittance may be, for example, 60% or less, may be 30% or less, or may be 10% or less.

The viscosity of the fine fibrous cellulose-containing composition slurry whose solid content concentration of the fine fibrous cellulose is adjusted to 0.4% by mass, which is measured by using a B-type viscometer under conditions of 23°C and a rotation speed of 3 rpm, is preferably $1.0\times10^4$mPa·s or less, more preferably $5.0\times10^3$mPa·s or less, further preferably $3.0\times10^3$mPa·s or less. In addition, the lower limit of the viscosity is not particularly limited, but is preferably $1.0\times10^2$ mPa·s or more, more preferably $3.0\times10^2$mPa·s or more, further preferably $5.0\times10^2$ mPa·s or more.

Here, in the measurement of the viscosity, for example, an analog viscometer T-LVT manufactured by BLOOKFIELD, which is a B-type viscometer, may be used. Measurement conditions: the fine fibrous cellulose-containing composition is diluted so that the solid content concentration of the fine fibrous cellulose becomes 0.4% by mass, and is stirred by a disperser at, for example, 1500 rpm for 5 min, and then is allowed to stand still for 24 h under an environment of 23°C and a relative humidity of 50%. Next, for example, at a liquid temperature of 23°C, measurement is performed at a viscometer rotation speed of 3 rpm, and 3 min after the start of the measurement, the viscosity value is set as the viscosity of the corresponding dispersion liquid.

It is desirable that the solvent at the time of the viscosity measurement contains at least water, and the occupying content of water in the whole solvent is preferably 50% by mass or more, more preferably 70% by mass or more, further preferably 90% by mass or more, particularly preferably 95% by mass or more, and may be 100% by mass.

The form of the fine fibrous cellulose-containing composition is not particularly limited, but examples thereof include a liquid substance such as a slurry, a solid substance such as a granular form, or a gel substance.

(Slurry Form)

When the fine fibrous cellulose-containing composition is a slurry, the content of the fine fibrous cellulose contained in the fine fibrous cellulose-containing composition is preferably 0.1% by mass or more and 5.0% by mass or less, more preferably 0.3% by mass or more and 3.0% by mass or less, further preferably 0.5% by mass or more and 3.0% by mass or less based on the total mass of the fine fibrous cellulose-containing composition. When the content of the fine fibrous cellulose is set within the range, properties included in the fine fibrous cellulose are easily exhibited.

When the fine fibrous cellulose-containing composition is a slurry, and the solvent contained in the fine fibrous cellulose-containing composition is water, the content of the corresponding solvent is preferably 60% by mass or more, more preferably 90% by mass or more, further preferably 97% by mass or more, and is preferably 99.9% by mass or less, more preferably 99.7% by mass or less, further preferably 99.5% by mass or less based on the total mass of the fine fibrous cellulose-containing composition. When the content of the solvent is set within the range, a suitable viscosity may be obtained.

(Solid Substance or Gel Substance)

When the fine fibrous cellulose-containing composition is a solid substance or a gel substance, the content of the fine fibrous cellulose contained in the fine fibrous cellulose-containing composition is preferably, for example, 5% by mass or more, more preferably 10% by mass or more, further preferably 15% by mass or more, particularly preferably 20% by mass or more based on the total mass of the fine fibrous cellulose-containing composition. In addition, when the fine fibrous cellulose-containing composition is a solid substance or a gel substance, the upper limit value of the content of the fine fibrous cellulose contained in the fine fibrous cellulose-containing composition is not particularly limited, but may be set to, for example, 99.5% by mass. When the content of the fine fibrous cellulose is set within the range, it is possible to obtain a fine fibrous cellulose-containing composition more excellent in the handleability.

When the fine fibrous cellulose-containing composition is a solid substance or a gel substance, the content of the solvent contained in the fine fibrous cellulose-containing composition is preferably, for example, 90% by mass or less, more preferably 85% by mass or less, further preferably 80% by mass or less, particularly preferably 70% by mass or less based on the total mass of the fine fibrous cellulose-containing composition. When the upper limit value of the content of the solvent is set within the range, it is possible to obtain a fine fibrous cellulose-containing composition excellent in the handleability. Meanwhile, the lower limit value of the content of the solvent contained in the fine fibrous cellulose-containing composition is not particularly limited, and may be, for example, 0% by mass. In the present embodiment, the content of the solvent contained in the fine fibrous cellulose-containing composition may be set to, for

example, 0.5% by mass or more based on the total mass of the fine fibrous cellulose-containing composition.

In addition, when the fine fibrous cellulose-containing composition is a solid substance or a gel substance, the fine fibrous cellulose-containing composition may contain, for example, a concentrating agent to be used in a concentration step to be described below. For example, when the concentrating agent has a metal component, the content of the metal component in the fine fibrous cellulose-containing composition is preferably 1.0% by mass or less, more preferably 0.3% by mass or less, further preferably 0.15% by mass or less, particularly preferably 0.1% by mass or less. In addition, when the concentrating agent has a metal component, the lower limit value of the content of the metal component in the fine fibrous cellulose-containing composition is not particularly limited, but may be set to, for example, 0.0001% by mass. When the content of the metal component is set within the range, it is possible to obtain a fine fibrous cellulose-containing composition excellent in re-dispersibility in a predetermined dispersion medium such as water.

When the fine fibrous cellulose-containing composition has a powder form (powder-grain substance), the fine fibrous cellulose-containing composition contains one or both of a powdery substance and a granular substance. Here, the powdery substance refers to those smaller than the granular substance. In general, the powdery substance refers to fine particles having a particle diameter of 1 nm or more and less than 0.1 mm. In addition, the granular substance refers to particles having a particle diameter of 0.1 mm or more and 10 mm or less. The particle diameter of the powder-grain substance may be measured and calculated by using, for example, a laser diffraction method. The measurement using the laser diffraction method may be performed by using, for example, a laser diffraction scattering-type particle diameter distribution measuring device (Microtrac3300EXII, Nikkiso Co., Ltd.).

The fine fibrous cellulose-containing composition as a solid substance or a gel substance may be used as, for example, a re-dispersed slurry obtained through re-dispersion in a solvent such as water. The type of the solvent used for obtaining such a re-dispersed slurry is not particularly limited, but examples thereof include water, an organic solvent, and a mixture of water and an organic solvent. Examples of the organic solvent include alcohols, polyhydric alcohols, ketones, ethers, esters, hydrocarbons, halogens, and an aprotic polar solvent. Examples of the alcohols include methanol, ethanol, isopropanol, n-butanol, and isobutylalcohol. Examples of the polyhydric alcohols include ethyleneglycol, propyleneglycol, and glycerin. Examples of the ketones include acetone, and methylethylketone (MEK). Examples of the ethers include diethylether, tetrahydrofuran, ethyleneglycolmonomethylether, ethyleneglycolmonoethylether, ethyleneglycolmono n-butylether, and propyleneglycolmonomethylether. Examples of the esters include ethyl acetate, and butyl acetate. Examples of the hydrocarbons include n-hexane, toluene, and xylene. Examples of the halogens include methylene chloride, trichloroethylene, and chloroform. Examples of the aprotic polar solvent include dimethylsulfoxide (DMSO), dimethylformamide (DMF), dimethylacetamide (DMAc), and N-methyl-2-pyrrolidinone (NMP).

(Sheet Form)

In the present invention, the fine fibrous cellulose-containing composition may have a sheet form.

In the present embodiment, for example, a sheet may be obtained by performing a sheet forming step to be described below by using the fine fibrous cellulose-containing composition as the above-described liquid substance.

The content of the fine fibrous cellulose in the sheet is preferably, for example, 0.5% by mass or more, more preferably 1% by mass or more, further preferably 5% by mass or more, particularly preferably 10% by mass or more based on the total mass of the sheet. Meanwhile, the upper limit value of the content of the fine fibrous cellulose in the sheet is not particularly limited, and may be 100% by mass or may be 95% by mass based on the total mass of the sheet.

In particular, from the viewpoint of obtaining a sheet excellent in the mechanical strength, the content of the fine fibrous cellulose in the sheet is preferably, for example, 60% by mass or more, more preferably 70% by mass or more, further preferably 80% by mass or more, particularly preferably 90% by mass or more.

In addition, from the viewpoint of allowing a large amount of other materials such as a water-soluble polymer to be contained in the sheet, it is preferable that the content of the fine fibrous cellulose in the sheet is, for example, 50% by mass or less.

The sheet may contain, for example, a water-soluble polymer. Accordingly, it is possible to further improve the transparency or the mechanical strength of the sheet. Examples of the water-soluble polymer include those described above.

The content of the water-soluble polymer in the sheet is preferably, for example, 1% by mass or more, more preferably 5% by mass or more, further preferably 10% by mass or more based on the total mass of the sheet. Accordingly, it is possible to obtain a sheet excellent in the transparency or the mechanical properties. Meanwhile, the content of the water-soluble polymer in the sheet is preferably set to, for example, 99.5% by mass or less based on the total mass of the sheet, and is more preferably 95% by mass or less.

The sheet may contain not only the fine fibrous cellulose, the enzyme-derived protein, the protease, or the chlorine-containing oxo acid or a salt thereof, and the water-soluble polymer, but also, for example, one type or two or more types selected from a surfactant, an organic ion, a coupling agent, an inorganic layered compound, an inorganic compound, a levelling agent, a preservative, a defoamer, organic particles, a lubricant, an antistatic agent, a UV protecting

agent, a dye, a pigment, a stabilizer, magnetic powder, an orientation accelerator, a plasticizer, a dispersant, and a cross-linking agent.

The sheet may contain a solvent. Examples of the solvent include those described above.

The content of the solvent in the sheet is preferably, for example, 0.5% by mass or more, more preferably 1% by mass or more, further preferably 5% by mass or more based on the total mass of the sheet. Accordingly, it is possible to impart flexibility to the sheet. Meanwhile, the content of the solvent in the sheet is preferably set to, for example, 25% by mass or less, more preferably 15% by mass or less based on the total mass of the sheet. Accordingly, a sheet having a good flexibility may be obtained.

The content of water in the sheet may be calculated by, for example, the following procedure. First, the humidity of a 100 mm-square sheet is adjusted for 24 h under conditions of a temperature of 23°C and a relative humidity of 50%, and then the mass $W_0$ of the sheet is measured. Then, the sheet is dried for 16 h in a constant temperature dryer of 105°C, and then, the mass $W_1$ of the sheet is measured. From the measured masses, the content of the solvent in the sheet is calculated according to the following formula (1).

$$\text{content of water in sheet} = 100 \times (1 - W_1/W_0) \qquad \text{Eq. (1)}$$

The tensile elastic modulus of the sheet is preferably, for example, 2.5 GPa or more, more preferably 3.0 GPa or more, further preferably 3.5 GPa or more. In addition, the upper limit value of the tensile elastic modulus of the sheet is not particularly limited, but may be set to, for example, 50 GPa or less.

Here, the tensile elastic modulus of the sheet is, for example, a value that is measured by using a tensile tester TENSILON (manufactured by A & D COMPANY) in accordance with JIS P 8113. When the tensile elastic modulus is measured, one whose humidity has been adjusted for 24 h at 23°C at a relative humidity of 50% is set as a test piece for measurement, and measurement is performed under conditions of 23°C and a relative humidity of 50%.

The haze of the sheet is preferably, for example, 2% or less, more preferably 1.5% or less, further preferably 1% or less. Meanwhile, the lower limit value of the haze of the sheet is not particularly limited, and may be, for example, 0%. Here, the haze of the sheet is, for example, a value that is measured by using a hazemeter (manufactured by MURAKAMI COLOR RESEARCH LABORATORY Co., Ltd., HM-150) in accordance with JIS K 7136:2006.

The total light transmittance of the sheet is preferably, for example, 85% or more, more preferably 90% or more, further preferably 91% or more. Meanwhile, the upper limit value of the total light transmittance of the sheet is not particularly limited, and may be, for example, 100%. Here, the total light transmittance of the sheet is, for example, a value that is measured by using a hazemeter (manufactured by MURAKAMI COLOR RESEARCH LABORATORY Co., Ltd., HM-150) in accordance with JIS K 7361:1997.

The thickness of the sheet is not particularly limited, but is preferably, for example, 5 μm or more, more preferably 10 μm or more, further preferably 20 μm or more. In addition, the upper limit value of the thickness of the sheet is not particularly limited, but may be set to, for example, 1,000 μm. The thickness of the sheet may be measured by, for example, a needle-type thickness gauge (manufactured by MAHR, Millitron 1202D).

The basis weight of the sheet is not particularly limited, but is preferably, for example, 10 $g/m^2$ or more, more preferably 20 $g/m^2$ or more, further preferably 30 $g/m^2$ or more. In addition, the basis weight of the sheet is not particularly limited, but is preferably, for example, 100 $g/m^2$ or less, more preferably 80 $g/m^2$ or less. Here, the basis weight of the sheet may be calculated in accordance with, for example, JIS P 8124.

The density of the sheet is not particularly limited, but is preferably, for example, 0.1 $g/cm^3$ or more, more preferably 0.5 $g/cm^3$ or more, further preferably 1.0 $g/cm^3$ or more. In addition, the density of the sheet is not particularly limited, but is preferably, for example, 5.0 $g/cm^3$ or less, more preferably 3.0 $g/cm^3$ or less. Here, the density of the sheet may be calculated by adjusting the humidity of a 50 mm-square sheet for 24 h under conditions of 23°C and 50%RH and then measuring the thickness and the mass of the sheet.

[Method of Producing Fine Fibrous Cellulose-Containing Composition]

In the present invention, it is desirable that the above-described fine fibrous cellulose-containing composition is produced by a method including the following steps 1 to 3 in this order.

step 1: a step of obtaining a fine fibrous cellulose-containing dispersion liquid by defibering fibrous cellulose in a solvent,
step 2: a step of adding an enzyme to the fine fibrous cellulose-containing dispersion liquid, and
step 3: a step of adding a chlorine-containing oxo acid or a salt thereof to the enzyme-treated fine fibrous cellulose-containing dispersion liquid.

In the present invention, the enzyme added in step 2 is preferably cellulase as described above, more preferably endoglucanase. In addition, when the enzyme is cellulase, the amount of the enzyme added is preferably 0.0001 U or more, more preferably 0.0005 U or more, further preferably 0.001 U or more based on 1 mg of the fine fibrous cellulose from the viewpoint of reducing the viscosity of the fine fibrous cellulose-containing composition, and is preferably 1.0 U or less, more preferably 0.1 U or less, further preferably 0.01 U or less from the viewpoint of suppressing an excessive reduction of the viscosity, and the viewpoint of the economy.

The addition amount of the chlorine-containing oxo acid or a salt thereof added in step 3 is preferably 0.0001 parts by mass or more, more preferably 0.0003 parts by mass or more, further preferably 0.001 parts by mass or more, yet more preferably 0.01 parts by mass or more, still more preferably 0.1 parts by mass or more based on 100 parts by mass of the fine fibrous cellulose from the viewpoint of allowing moderate viscosity reduction to be progressed, and inactivating the enzyme and also obtaining a sufficient viscosity reducing effect, and is preferably 10 parts by mass or less, more preferably 5 parts by mass or less, further preferably 3 parts by mass or less, still more preferably 1 part by mass or less.

Here, as described in the method of producing the fine fibrous cellulose, it is desirable that the step of obtaining the fine fibrous cellulose-containing dispersion liquid in step 1 includes an ionic substituent introducing step of introducing an ionic substituent into a cellulose-containing fiber raw material, a washing step, an alkali treatment step (neutralization step), and a defibering step in this order, and instead of the washing step, or in addition to the washing step, an acid treatment step may be included. The ionic substituent introducing step may be exemplified with a phosphoric acid group introducing step and a carboxy group introducing step. Therefore, in the present invention, it is desirable that the fine fibrous cellulose in the fine fibrous cellulose-containing dispersion liquid obtained in step 1 has the ionic substituent.

In step 2, the enzyme is added to the fine fibrous cellulose-containing dispersion liquid. The addition method is not particularly limited, but from the viewpoint of obtaining a high enzyme activity, the enzyme optimal pH$\pm$3 is preferable, the enzyme optimal pH$\pm$2 is more preferable, and the enzyme optimal pH$\pm$1 is further preferable.

When the enzyme is cellulase, pH of the dispersion liquid to which the enzyme is added is preferably 3 or more, more preferably 4 or more, further preferably 5 or more, and is preferably 12 or less, more preferably 11 or less, further preferably 10 or less.

In addition, the temperature of the dispersion liquid at the time of treatment with the enzyme in step 2 depends on the optimal temperature of the enzyme, but is preferably, for example, 10°C or more, more preferably 15°C or more, further preferably 20°C or more from the viewpoint of obtaining a high enzyme activity, and is preferably 80°C or less, more preferably 70°C or less, further preferably 60°C or less.

The time for the treatment with the enzyme in step 2 may be properly selected according to the temperature, the addition amount of the enzyme, etc. but is preferably 3 min or more, more preferably 10 min or more, further preferably 15 min or more, and is preferably 24h or less, more preferably 12 h or less, further preferably 8 h or less from the viewpoint of productivity or the like.

In step 3, the chlorine-containing oxo acid or a salt thereof is added to the enzyme-treated fine fibrous cellulose-containing dispersion liquid. The addition method is not particularly limited, but it is desirable that addition is performed such that the addition amount of the chlorine-containing oxo acid or a salt thereof becomes 0.0001 parts by mass or more and 10 mass or less based on 100 parts by mass of the fine fibrous cellulose.

It is desirable that the addition of the chlorine-containing oxo acid or a salt thereof is performed with stirring such that the chlorine-containing oxo acid or a salt thereof is not present at a high concentration in the dispersion liquid.

The fibrous cellulose-containing composition of the present invention may be used as, for example, a thickener for various purposes. In addition, a use as a reinforcing material through mixing with a resin or an emulsion is possible, and various sheets may be prepared through film formation using a slurry of the fine fibrous cellulose-containing composition. The sheets are suitable for uses for light transmissive substrates such as various display devices, and various solar cells. In addition, the sheets are suitable for uses for electronic device boards, home appliance parts, window materials, interior materials, and exterior materials in various vehicles or buildings, packaging materials and the like. Further the sheets are suitable for not only yarns, filters, woven fabrics, cushioning materials, sponges, abrasives and the like, but also purposes in which the sheet itself is used as a reinforcing material.

[Examples]

Hereinafter, the features of the present invention will be more specifically described with reference to Examples and Comparative examples. Materials, use amounts, ratios, processing contents, processing procedures and the like described in Examples below may be properly changed as long as they do not deviate from the gist of the present invention. Therefore, the scope of the present invention should not be construed as limited by specific examples described below.

<Production Example 1>

As raw material pulp, needle leaved tree kraft pulp manufactured by Oji Paper (solid content 93% by mass, basis weight 208 g/$m^2$, sheet form, Canada standard freeness (CSF) measured in accordance with JIS P 8121 after disaggregation is 700 mL) was used.

On the raw material pulp, a phosphorylation treatment was performed as follows. First, a mixed aqueous solution of ammonium dihydrogen phosphate and urea was added to 100 parts by mass (absolute dry mass) of the raw material pulp, and preparation was performed for 45 parts by mass of ammonium dihydrogen phosphate, 120 parts by mass of urea, and 150 parts by mass of water. Then, chemical-impregnated pulp was obtained. Next, the obtained chemical-impregnated pulp was heated by a hot air dryer of 165°C for 200 sec, and a phosphoric acid group was introduced into cellulose in the pulp to obtain phosphorylated pulp.

Next, a washing treatment was performed on the obtained phosphorylated pulp. The washing treatment was performed by repeating an operation in which a pulp dispersion liquid that was obtained by pouring 10 L of ion-exchanged water into 100 g (absolute dry mass) of the phosphorylated pulp was stirred such that the pulp was uniformly dispersed, and then, filtering and dehydration were performed. The point in time when the electrical conductivity of the filtrate became 100 μS/cm or less was set as the end point of washing.

Next, an alkali treatment (neutralization treatment) was performed on the washed phosphorylated pulp in the following manner. First, the washed phosphorylated pulp was diluted with 10 L of ion-exchanged water, and then was stirred while a 1 N sodium hydroxide aqueous solution was added thereto little by little so as to obtain a phosphorylated pulp slurry with pH of 12 or more and 13 or less. Next, the corresponding phosphorylated pulp slurry was dehydrated to obtain alkali-treated (neutralized) phosphorylated pulp.

Then, the washing treatment was performed on the neutralized phosphorylated pulp. On the phosphorylated pulp obtained in this manner, an infrared absorption spectrum was measured by using FT-IR. As a result, absorption based on a phosphoric acid group was observed around 1230 $cm^{-1}$, and then it was confirmed that the phosphoric acid group was added to the pulp. In addition, the obtained phosphorylated pulp was provided, and analyzed by an X-ray diffraction device, and as a result, typical peaks were confirmed at two positions around $2\theta$=14° or more and 17° or less and around $2\theta$=22° or more and 23° or less, and it was confirmed that cellulose I-type crystals were included.

(Defibering Process)

Ion-exchanged water was added to the obtained phosphorylated pulp to prepare a slurry with a solid content concentration of 2% by mass. This slurry was treated with a wet pulverizing device (manufactured by Sugino Machine limited, STAR BURST) at a pressure of 200 MPa four times, to obtain a fine fibrous cellulose dispersion liquid (A) containing fine fibrous cellulose.

Through X-ray diffraction, it was confirmed that the fine fibrous cellulose maintained the cellulose I-type crystals.

In addition, when the fiber width of the fine fibrous cellulose was measured by using a transmission type electron microscope, the result was 3 to 5 nm. An amount of phosphoric acid groups (an amount of strong acid groups) measured by a measurement method to be described below was 1.45 mmol/g.

(Measurement of Amount of Phosphoric Acid Groups)

The amount of phosphoric acid groups in fine fibrous cellulose was measured by performing a treatment with an ion-exchanged resin on a fine fibrous cellulose-containing slurry that was produced by diluting a fine fibrous cellulose dispersion liquid containing target fine fibrous cellulose with ion-exchanged water (a content of 0.2% by mass), and then, performing titration using alkali.

The treatment with the ion-exchanged resin was performed by adding a strong acid ion-exchanged resin (Amberjet 1024; manufactured by ORGANO CORPORATION, conditioned) with a volume of 1/10 to the fine fibrous cellulose-containing slurry, and performing shaking for 1 h. Then, through pouring on a mesh having a mesh size of 90 μm, the resin was separated from the slurry.

In addition, the titration using alkali was performed by measuring the change in the value of the electrical conductivity indicated by the slurry while adding 50 μL of a 0.1 N sodium hydroxide aqueous solution every 30 seconds to the fine fibrous cellulose-containing slurry that had been subjected to the treatment with the ion-exchanged resin. The amount of the phosphoric acid groups (mmol/g) was calculated by dividing the amount of required alkali (mmol) in a region in the measurement result corresponding to the first region illustrated in Fig. 1, by the solid content (g) in the slurry as a titration target.

<Production Example 2>

As raw material pulp, needle leaved tree kraft pulp (undried) manufactured by Oji Paper Co., Ltd. was used. On the raw material pulp, an alkali TEMPO oxidation treatment was performed as follows.

First, the raw material pulp equivalent to 100 parts by mass (dry mass), 1.6 parts by mass of TEMPO (2,2,6,6-tetramethylpiperidine-1-oxyl), and 10 parts by mass of sodium bromide were dispersed in 10,000 parts by mass of water. Then, a sodium hypochlorite aqueous solution of 13% by mass was added to 1.0 g of the pulp up to 3.8 mmol and the reaction was started. During the reaction, a 0.5 M sodium hydroxide aqueous solution was added dropwise so that pH was maintained at 10 or more and 10.5 or less, and then a point in time when no change occurred in pH was considered as the end of the reaction.

Next, a washing treatment was performed on the obtained TEMPO oxidized pulp. The washing treatment was performed by repeating an operation in which a dehydrated sheet was obtained by dehydrating the TEMPO oxidized pulp slurry, and was uniformly dispersed through stirring after 5,000 parts by mass of ion-exchanged water was poured, and then filtration and dehydration were performed. The point in time when the electrical conductivity of the filtrate became 100 μS/cm or less was set as the end point of washing.

On the dehydrated sheet, an additional oxidation treatment of a remaining aldehyde group was performed as follows. The dehydrated sheet equivalent to 100 parts by mass (dry mass) was dispersed in 10,000 parts by mass of 0.1 mol/L acetic acid buffer (pH 4.8). Then, 113 parts by mass of 80% sodium chlorite was added, and after immediate sealing, a reaction was performed at room temperature for 48 h while stirring was performed by using a magnetic stirrer at 500 rpm. Then, a pulp slurry was obtained.

Next, a washing treatment was performed on the obtained TEMPO oxidized pulp which had been subjected to the additional oxidation. The washing treatment was performed by repeating an operation in which a dehydrated sheet was obtained by dehydrating the additionally oxidized pulp slurry, and was uniformly dispersed through stirring after 5,000 parts by mass of ion-exchanged water was poured, and then filtration and dehydration were performed. The point in time when the electrical conductivity of the filtrate became 100 μS/cm or less was set as the end point of washing.

In regard to the TEMPO oxidized pulp obtained in this manner, an amount of carboxy groups measured by a measurement method to be described below was 1.30 mmol/g.

In addition, the obtained TEMPO oxidized pulp was provided, and analyzed by an X-ray diffraction device, and as a result, typical peaks were confirmed at two positions around 2θ=14° or more and 17° or less and around 2θ=22° or more and 23° or less, and it was confirmed that cellulose I-type crystals were included.

(Defibering Process)

Ion-exchanged water was added to the obtained TEMPO oxidized pulp to prepare a slurry with a solid content concentration of 2% by mass. This slurry was treated with a wet pulverizing device (manufactured by Sugino Machine limited, STAR BURST) at a pressure of 200 MPa four times, to obtain a fine fibrous cellulose dispersion liquid (B) containing fine fibrous cellulose.

Through X-ray diffraction, it was confirmed that the fine fibrous cellulose maintained the cellulose I-type crystals.

In addition, when the fiber width of the fine fibrous cellulose was measured by using a transmission type electron microscope, the result was 3 to 5 nm. An amount of carboxy groups measured by a measurement method to be described below was 1.30 mmol/g.

(Measurement of Amount of Carboxy Groups)

The amount of carboxy groups in fine fibrous cellulose was measured by performing a treatment with an ion-exchanged resin on a fine fibrous cellulose-containing slurry that was produced by diluting a fine fibrous cellulose dispersion liquid containing target fine fibrous cellulose with ion-exchanged water (a content of 0.2% by mass), and then, performing titration using alkali.

The treatment with the ion-exchanged resin was performed by adding a strong acid ion-exchanged resin (Amberjet 1024; manufactured by ORGANO CORPORATION, conditioned) with a volume of 1/10 to the fine fibrous cellulose-containing slurry, and performing shaking for 1 h. Then, through pouring on a mesh having a mesh size of 90 μm, the resin was separated from the slurry.

In addition, the titration using alkali was performed by measuring the change in the value of the electrical conductivity indicated by the slurry while adding 50 μL of a 0.1 N sodium hydroxide aqueous solution every 30 seconds to the fine fibrous cellulose-containing slurry that had been subjected to the treatment with the ion-exchanged resin. The amount of the carboxy groups (mmol/g) was calculated by dividing the amount of required alkali (mmol) in a region in the measurement result corresponding to the first region illustrated in Fig. 2, by the solid content (g) in the slurry as a titration target.

<Example 1>

The concentration of the fine fibrous cellulose dispersion liquid (A) was adjusted to 2% by mass, and 5.0 parts by mass (0.0042 U based on 1 mg of the fine fibrous cellulose) of cellulase (ECOPULP, manufactured by AB Enzymes) diluted 1,000 times with ion-exchanged water was added per 100 parts by mass of the fine fibrous cellulose. Then, stirring was performed at a temperature of 50°C for 30 min. pH of the fine fibrous cellulose-containing dispersion liquid at the time of enzyme addition was 8.8. Next, a sodium hypochlorite solution with an effective chlorine concentration of 12% (sodium hypochlorite concentration 12% by mass) was added such that the sodium hypochlorite became 0.15 parts by mass based on 100 parts by mass of the fine fibrous cellulose to which the enzyme was not added. Through stirring at 40°C for 15 min, a composition containing the fine fibrous cellulose, an enzyme-derived protein, and the sodium hypochlorite was obtained.

<Example 2>

The addition amount of the sodium hypochlorite solution with the effective chlorine concentration of 12% in Example 1 was changed such that the sodium hypochlorite became 10 parts by mass based on 100 parts by mass of the fine fibrous cellulose to which the enzyme was not added. In regard to all others, a test was performed in the same manner as in Example 1.

<Example 3>

Except that 0.5 parts by mass (0.00042 U based on 1 mg of the fine fibrous cellulose) of cellulase (product name of cellulase: ECOPULP, manufactured by AB Enzymes) diluted 1,000 times with ion-exchanged water was added per 100 parts by mass of the fine fibrous cellulose in Example 1, in regard to all others, a test was performed in the same manner as in Example 1.

<Example 4>

The fine fibrous cellulose dispersion liquid (B) (2% by mass) was used in Example 1. In regard to all others, a test was performed in the same manner as in Example 1.

<Example 5>

The concentration of the fine fibrous cellulose dispersion liquid (A) was adjusted to 2% by mass, and 5.0 parts by mass (0.0042U based on 1 mg of the fine fibrous cellulose) of cellulase (ECOPULP, manufactured by AB Enzymes) diluted 1,000 times with ion-exchanged water was added per 100 parts by mass of the fine fibrous cellulose. Then, stirring was performed at a temperature of 50°C for 30 min. pH of the fine fibrous cellulose-containing dispersion liquid at the time of enzyme addition was 8.8. Next, on the dispersion liquid, addition was performed such that a protease became 0.01 U per 1 mg of the fine fibrous cellulose. Through stirring, a composition containing the fine fibrous cellulose and the protease was obtained. This was stirred at 40°C for 15 min to obtain a composition containing the fine fibrous cellulose, and an enzyme-derived protein.

<Example 6>

Except that the addition amount of the protease in Example 5 was changed to 0.5 U per 1 mg of the fine fibrous cellulose, in regard to all others, a test was performed in the same manner as in Example 5.

<Example 7>

Except that 0.5 parts by mass (0.00042 U based on 1 mg of the fine fibrous cellulose) of cellulase (product name of cellulase: ECOPULP, manufactured by AB Enzymes) diluted 1,000 times with ion-exchanged water was added per 100 parts by mass of the fine fibrous cellulose in Example 5, in regard to all others, a test was performed in the same manner as in Example 5.

<Example 8>

The fine fibrous cellulose dispersion liquid (B) (2% by mass) was used in Example 5. In regard to all others, a test was performed in the same manner as in Example 5.

<Comparative Example 1>

In Example 1, cellulase (cellulase product name ECOPULP, manufactured by AB Enzymes) diluted 1,000 times with ion-exchanged water and a sodium hypochlorite solution were not added. In regard to all others, a test was performed in the same manner as in Example 1.

<Comparative Example 2>

In Example 4, cellulase (cellulase product name: ECOPULP, manufactured by AB Enzymes) diluted 1,000 times with ion-exchanged water and a sodium hypochlorite solution were not added. In regard to all others, a test was performed in the same manner as in Example 4.

<Comparative Example 3>

In Example 1, a sodium hypochlorite solution was not added. In regard to all others, a test was performed in the same manner as in Example 1.

(Measurement of Fiber Width of Fine Fibrous Cellulose)

The fiber width of the fine fibrous cellulose was measured by the following method.
A supernatant of the fine fibrous cellulose dispersion liquid obtained through treatment with a wet pulverizing device was diluted with water such that the concentration of the fine fibrous cellulose became 0.01% by mass or more and 0.1% by mass or less, and then was added dropwise to a hydrophilized carbon grid film. This was dried, dyed with uranyl acetate, and observed with a transmission type electron microscope (manufactured by JEOL LTD., JEOL-2000EX).

(Measurement of Viscosity of Fine Fibrous Cellulose Dispersion Liquid)

The viscosity of the fine fibrous cellulose dispersion liquid was measured as follows. First, the fine fibrous cellulose dispersion liquid was diluted with ion-exchanged water such that the solid content concentration became 0.4% by mass, and then was stirred by a disperser at 1500 rpm for 5 min. The dispersion liquid as a measurement target was allowed to stand still for 24 h under an environment of 23°C and a relative humidity of 50% before the measurement. Then, the viscosity of the dispersion liquid obtained in this manner was measured by using a B-type viscometer (manufactured by BLOOKFIELD, an analog viscometer T-LVT). Under the measurement condition of a rotation speed of 3 rpm, 3 min after the start of measurement, the viscosity value was set as the viscosity of the corresponding dispersion liquid. In addition, the liquid temperature of the dispersion liquid at the time of measurement was 23°C.
A measurement limit in the viscometer is 100 mPa·s Even for a viscosity equal to or less than the measurement limit, measurement itself is possible, but the quantitativeness tends to be lost.
The viscosity after 72 h since preparation of the fine fibrous cellulose dispersion liquid, and the viscosity after 168 h were measured.

(Measurement on Specific Viscosity and Degree of Polymerization of Cellulose Fibers)

The specific viscosity and the degree of polymerization of cellulose fibers were measured according to Tappi T230. That is, after measurement was performed on a viscosity (referred to as $\eta_1$ (unit; cP)) measured when cellulose fibers as a measurement target were dispersed in a dispersion medium, and a blank viscosity (referred to as $\eta_0$ (unit; cP)) measured by only a dispersion medium, a specific viscosity ($\eta_{sp}$), and an intrinsic viscosity ([η] (unit; mL/g)) were measured according to the following formulas.

$$\eta_{sp}=(\eta_1/\eta_0)-1$$

$$[\eta]=\eta_{sp}/(c(1+0.28\times\eta_{sp}))$$

Here, c in the formula indicates a concentration (g/mL) of the cellulose fibers at the time of the viscosity measurement.
Further, the degree of polymerization (DP) of the cellulose fibers was calculated from the following formula.

$$DP=1.75\times[\eta]$$

This degree of polymerization is an average degree of polymerization measured according to a viscosity method, and thus may be referred to as a "viscosity average polymerization degree."

(Determination on Presence/Absence of Discoloration of Fine Fibrous Cellulose Dispersion Liquid)

72 h after preparation of 0.4% by mass of the fine fibrous cellulose dispersion liquid, presence/absence of discoloration was determined through visual observation.

Table 1

| | Example | | | | | | | | Comparative Example | | |
|---|---|---|---|---|---|---|---|---|---|---|---|
| | 1 | 2 | 3 | 4 | 5 | 6 | 7 | 8 | 1 | 2 | 3 |
| Substituent introduction method | Phosphor-rylated | Phosphor-rylated | Phosphor-rylated | TEMPO oxidized | Phosphor-rylated | Phosphor-rylated | Phosphor-rylated | TEMPO oxidized | Phosphor-rylated | TEMPO oxidized | Phospho-rylated |
| Addition amount (parts by mass) of enzyme per 100 parts by mass of fine fibrous cellulose | $5.0\times10^{-3}$ | $5.0\times10^{-3}$ | $5.0\times10^{-4}$ | $5.0\times10^{-3}$ | $5.0\times10^{-3}$ | $5.0\times10^{-3}$ | $5.0\times10^{4}$ | $5.0\times10^{-3}$ | - | - | $5.0\times10^{-3}$ |
| Addition amount (U/mg) of enzyme per 1 mg of fine fibrous cellulose | 0.0042 | 0.0042 | 0.00042 | 0.0042 | 0.0042 | 0.0042 | 0.00042 | 0.0042 | - | - | 0.0042 |
| Addition amount (parts by mass) of NaClO per 100 parts by mass of fine fibrous cellulose | 0.15 | 10 | 0.15 | 0.15 | - | - | - | - | - | - | - |
| Content (parts by mass) of NaClO based on 100 parts by mass of enzyme- derived protein | 3,000 | 200,000 | 30,000 | 3,000 | - | - | - | - | - | - | - |
| Addition amount (U/mg) of protease per 1 mg of fine fibrous cellulose | - | - | - | - | 0.01 | 0.5 | 0.01 | 0.01 | - | - | - |

(continued)

| | Example | | | | | | | | Comparative Example | | |
|---|---|---|---|---|---|---|---|---|---|---|---|
| | 1 | 2 | 3 | 4 | 5 | 6 | 7 | 8 | 1 | 2 | 3 |
| Content (U/mg) of protease per 1 mg of enzyme-derived protein | - | | - | - | 200 | 10,000 | 2,000 | 200 | - | - | - |
| Viscosity (mPa·s) after 72h since preparation | 980 | 720 | 1,190 | 770 | 1,050 | 1,000 | 1,230 | 880 | 11,250 | 18,680 | 1,510 |
| Viscosity (mPa·s) after 168h since preparation | 960 | 700 | 1,180 | 740 | 1,030 | 980 | 1,190 | 860 | 11,180 | 18,550 | ND |
| Degree of polymerization | 298 | 281 | 326 | 288 | 305 | 300 | 335 | 295 | 510 | 501 | 181 |
| Discoloration | None | None | None | None | None | None | None | None | None | None | None |

As noted in Examples in the table, in Comparative Examples 1 and 2 in which an enzyme-derived protein and a protease or a chlorine-containing oxo acid or a salt thereof are not contained, the viscosities after 72 h were 11,250 mPa·s and 18,680 mPa·s in the fine fibrous cellulose-containing dispersion liquids, but were about 10% or less of these in the fine fibrous cellulose dispersion liquids in Examples 1 to 8. Since the purpose of this application is to precisely control the viscosity of the fine fibrous cellulose composition through simple steps, it can be thought that Examples 1 to 8 achieved the purposes.

In addition, since no discoloration of the compositions was confirmed in Examples 1 to 8, a property such as high transparency or colorlessness, which is one of characteristics of the fine fibrous cellulose, was not impaired.

Meanwhile, in Comparative Example 3, at a point in time when 72 h elapsed after preparation and a point in time when 168 h elapsed, the viscosity was largely reduced. It may be thought that this is because the enzyme was not decomposed or denatured, and thus, the enzyme continued to function even while the composition was standing still, and the decomposition of the cellulose was excessively progressed. Since the expected viscosity could not be maintained for a certain time, Comparative Example 3 was not suitable for the purpose of the present invention.

Industrial Applicability

According to the present invention, it is possible to provide a fine fibrous cellulose composition that is simply and precisely adjusted to an arbitrary viscosity. In order to adjust the viscosity of fine fibrous cellulose, in a conventional method, a further defibering process by a defibrator is required. This method requires a long time because a strict viscosity adjustment is difficult and the procedure is also complicated.

The present invention solves these problems, and thus uses of fine fibrous cellulose in various purposes such as thickeners for paints, cosmetics or foods or composites with resins can be expected.

## Claims

1. A fine fibrous cellulose-containing composition comprising fine fibrous cellulose having a fiber width of 1,000 nm or less, a protein derived from an enzyme, and a protease or a chlorine-containing oxo acid or a salt thereof.

2. The fine fibrous cellulose-containing composition according to claim 1, wherein the enzyme is a cellulase.

3. The fine fibrous cellulose-containing composition according to claim 1 or 2, wherein a degree of polymerization of the fine fibrous cellulose is 430 or less.

4. The fine fibrous cellulose-containing composition according to any one of claims 1 to 3, wherein the fine fibrous cellulose has an ionic substituent, and a content of the ionic substituent is 0.60 mmol/g or more with respect to the fine fibrous cellulose.

5. The fine fibrous cellulose-containing composition according to claim 4, wherein the ionic substituent is at least one selected from a carboxymethyl group, a sulfonic acid group, a group derived from the sulfonic acid group, a phosphoric acid group, a group derived from the phosphoric acid group, a carboxy group, and a group derived from the carboxy group.

6. The fine fibrous cellulose-containing composition according to any one of claims 1 to 5, wherein the fine fibrous cellulose-containing composition contains a chlorine-containing oxo acid or a salt thereof, and a content of the chlorine-containing oxo acid or a salt thereof is 0.0001 parts by mass or more and 10 parts by mass or less based on 100 parts by mass of the fine fibrous cellulose.

7. The fine fibrous cellulose-containing composition according to any one of claims 1 to 6, wherein the fine fibrous cellulose-containing composition contains a chlorine-containing oxo acid or a salt thereof, and contains 10 parts by mass or more and 1,000,000 parts by mass or less of the chlorine-containing oxo acid or a salt thereof based on 100 parts by mass of the protein derived from the enzyme.

8. The fine fibrous cellulose-containing composition according to any one of claims 1 to 7, wherein the fine fibrous cellulose-containing composition contains a chlorine-containing oxo acid or a salt thereof, and the chlorine-containing oxo acid or a salt thereof includes at least one selected from the group consisting of sodium hypochlorite, potassium hypochlorite, and calcium hypochlorite.

**9.** The fine fibrous cellulose-containing composition according to any one of claims 1 to 5, wherein the fine fibrous cellulose-containing composition contains a protease, and a content of the protease is 0.0002 U or more and 20 U or less based on 1 mg of the fine fibrous cellulose.

**10.** The fine fibrous cellulose-containing composition according to any one of claims 1 to 5, and 9, wherein the fine fibrous cellulose-containing composition contains a protease, and contains 1 U or more and 1,000,000 U or less of the protease based on 1 mg of the protein derived from the enzyme.

**11.** The fine fibrous cellulose-containing composition according to any one of claims 1 to 10, wherein when a solid content concentration of the fine fibrous cellulose is 0.4% by mass, a composition viscosity is $1.0\times10^4$ mPa·s or less as measured by using a B-type viscometer at 23°C through rotation for 3 min at a rotation speed of 3 rpm.

**12.** The fine fibrous cellulose-containing composition according to any one of claims 1 to 11, which has a slurry form.

**13.** The fine fibrous cellulose-containing composition according to any one of claims 1 to 10, which has a powder form.

**14.** The fine fibrous cellulose-containing composition according to any one of claims 1 to 10, which has a sheet form.

**15.** A method of producing a composition containing fine fibrous cellulose, the method comprising, in this order:

defibering fibrous cellulose in a solvent to obtain a fine fibrous cellulose-containing dispersion liquid;
adding an enzyme to the fine fibrous cellulose-containing dispersion liquid; and
adding a protease, or a chlorine-containing oxo acid or a salt thereof to the fine fibrous cellulose-containing dispersion liquid treated with the enzyme.

**16.** The method of producing the fine fibrous cellulose-containing composition according to claim 15, wherein the enzyme is a cellulase, and an addition amount of the cellulase is 0.0001 U or more and 1.0 U or less based on 1 mg of the fine fibrous cellulose.

**17.** The method of producing the fine fibrous cellulose-containing composition according to claim 15 or 16, wherein the fibrous cellulose has an ionic substituent.

[Fig. 1]

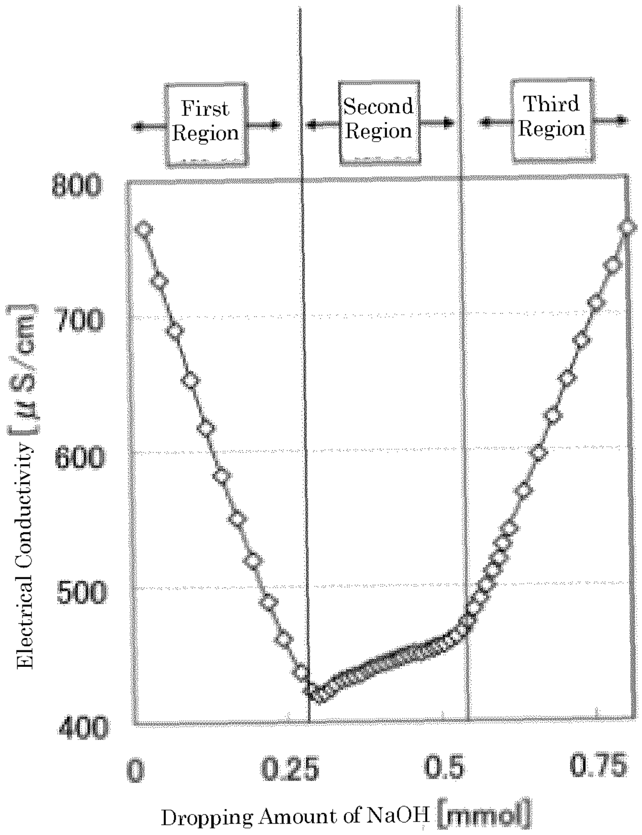
First Region
Second Region
Third Region
800
700
600
500
400
Electrical Conductivity [μS/cm]
0
0.25
0.5
0.75
Dropping Amount of NaOH [mmol]

[Fig. 2]

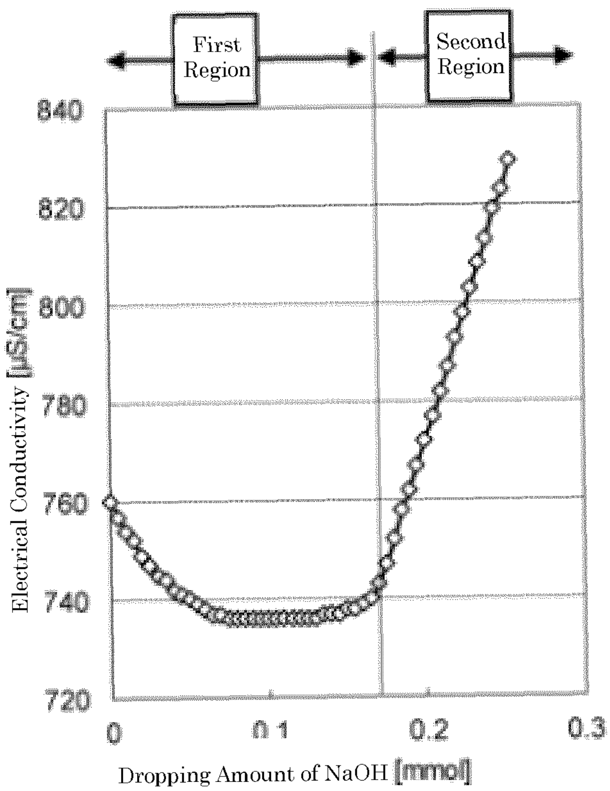
First Region
Second Region
840
820
800
780
760
740
720
Electrical Conductivity [μS/cm]
0
0.1
0.2
0.3
Dropping Amount of NaOH [mmol]

## INTERNATIONAL SEARCH REPORT

International application No.
PCT/JP2019/041876

A. CLASSIFICATION OF SUBJECT MATTER
C12P 19/04(2006.01)i; C08K 3/24(2006.01)i; C08L 1/02(2006.01)i; C08L 89/00(2006.01)i; D21H 11/20(2006.01)i
FI: C12P19/04 Z; C08K3/24; C08L1/02; C08L89/00; D21H11/20

According to International Patent Classification (IPC) or to both national classification and IPC

B. FIELDS SEARCHED

Minimum documentation searched (classification system followed by classification symbols)
C12P19/04; C08K3/24; C08L1/02; C08L89/00; D21H11/20

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| A | JP 2015-221844 A (TOPPAN PRINTING CO., LTD.) 10.12.2015 (2015-12-10) | 1-17 |
| A | JP 2013-241586 A (MITSUBISHI CHEMICAL CORP.) 05.12.2013 (2013-12-05) | 1-17 |
| A | JP 2009-298972 A (KAO CORP.) 24.12.2009 (2009-12-24) | 1-17 |
| A | JP 2013-110987 A (NATIONAL INSTITUTE OF ADVANCED INDUSTRIAL SCIENCE AND TECHNOLOGY) 10.06.2013 (2013-06-10) | 1-17 |
| A | WO 2012/043103 A1 (NIPPON PAPER INDUSTRIES CO., LTD.) 05.04.2012 (2012-04-05) | 1-17 |

☒ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 January 2020 (06.01.2020) | 21 January 2020 (21.01.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/041876

C (Continuation). DOCUMENTS CONSIDERED TO BE RELEVANT

| Category* | Citation of document, with indication, where appropriate, of the relevant passages | Relevant to claim No. |
|---|---|---|
| A | WO 2011/118748 A1 (NIPPON PAPER INDUSTRIES CO., LTD.) 29.09.2011 (2011-09-29) | 1-17 |
| A | WO 2011/118746 A1 (NIPPON PAPER INDUSTRIES CO., LTD.) 29.09.2011 (2011-09-29) | 1-17 |
| A | WO 2010/116826 A1 (NIPPON PAPER INDUSTRIES CO., LTD.) 14.10.2010 (2010-10-14) | 1-17 |
| A | JP 2012-111849 A (OJI PAPER CO., LTD.) 14 June 2012 (2012-06-14) | 1-17 |
| A | 林 徳子 et al., セルロースの酵素による微細化法, Cellulose Commun., 2009, 16(2), pp.73-78 entire text, non-official translation (HAYASHI, Noriko et al., “Microstructural Refinement Method by Cellulose enzymes”) | 1-17 |
| P,A | WO 2019/021866 A1 (OJI HOLDINGS CORPORATION) 31.01.2019 (2019-01-31) | 1-17 |

Form PCT/ISA/210 (continuation of second sheet) (January 2015)

**INTERNATIONAL SEARCH REPORT**
Information on patent family members

International application No.
PCT/JP2019/041876

| Patent Documents referred in the Report | Publication Date | Patent Family | Publication Date |
|---|---|---|---|
| JP 2015-221844 A | 10 Dec. 2015 | (Family: none) | |
| JP 2013-241586 A | 05 Dec. 2013 | (Family: none) | |
| JP 2009-298972 A | 24 Dec. 2009 | (Family: none) | |
| JP 2013-110987 A | 10 Jun. 2013 | (Family: none) | |
| WO 2012/043103 A1 | 05 Apr. 2012 | JP 2013-256546 A | |
| WO 2011/118748 A1 | 29 Sep. 2011 | (Family: none) | |
| WO 2011/118746 A1 | 29 Sep. 2011 | (Family: none) | |
| WO 2010/116826 A1 | 14 Oct. 2010 | JP 2010-236109 A<br>JP 2010-235679 A<br>JP 2010-235681 A<br>JP 2010-236106 A<br>JP 2010-275659 A | |
| JP 2012-111849 A | 14 Jun. 2012 | (Family: none) | |
| WO 2019/021866 A1 | 31 Jan. 2019 | JP 2019-26651 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018090949 A


### Non-patent literature cited in the description


- **SEAGAL et al.** *Textile Research Journal,* 1959, vol. 29, 786